# EUROPÄISCHE PATENTSCHRIFT

(11) **EP 3 821 828 B1**
(45) Veröffentlichungstag und Bekanntmachung des Hinweises auf die Patenterteilung: **26.03.2025**
(21) Anmeldenummer: 19208962.1
(22) Anmeldetag: 13.11.2019
(51) Int. Cl.: A61B 17/14, A61B 17/16, A61B 17/32, A61M 3/02, A61B 17/00

(54) **DRUCKGASMOTOR UND VERFAHREN ZUM BETREIBEN EINES DRUCKGASMOTORS**
COMPRESSED GAS MOTOR AND METHOD FOR OPERATING A COMPRESSED GAS MOTOR
MOTEUR À GAZ COMPRIMÉ ET PROCÉDÉ DE FONCTIONNEMENT D'UN MOTEUR À GAZ COMPRIMÉ

(43) Veröffentlichungstag der Anmeldung: 19.05.2021
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Schultheiss & Sterzel Patentanwälte PartG mbB

(56) Entgegenhaltungen:
- WO-A1-01/43651
- DE-B3- 102010 046 057
- US-A- 5 924 602
- US-B2- 6 736 292

## Beschreibung

Die Erfindung betrifft einen Druckgasmotor, ein chirurgisches Antriebssystem mit einem solchen Druckgasmotor, ein medizinisches Lavage-System zum Debridement von Weichgewebe und/oder Knochengewebe aufweisend einen solchen Druckgasmotor und eine medizinische Vorrichtung zum Bürsten, Raspeln oder Sägen von Weichgewebe und/oder Knochengewebe mit einem solchen Druckgasmotor sowie ein Verfahren zum Betreiben eines Druckgasmotors. Der Druckgasmotor ist zum Antrieb von medizinischen Vorrichtungen für das Lavagieren und das Debridement von Weich- und Knochengewebe geeignet. Die mit dem Druckgasmotor angetriebenen medizinischen Vorrichtungen sind vorzugsweise aus hygienischen Gründen zum einmaligen Gebrauch bestimmt.

Gegenstand der Erfindung sind insbesondere ein vereinfachter Druckgasmotor und ein chirurgisches also medizinisches Antriebssystem mit dem vereinfachten Druckgasmotor. Der vereinfachte Druckgasmotor und das chirurgische Antriebssystem bestehen im Wesentlichen aus Kunststoff und sind für die einmalige Verwendung bestimmt und geeignet.

In der orthopädischen Chirurgie müssen leider in einem gewissen Umfang septische Revisionen von mit Mikroorganismen infizierten Gelenkendoprothesen vorgenommen werden. Dabei werden die infizierten Gelenkendoprothesen explantiert und das infizierte beziehungsweise nekrotische Gewebe abgetragen. Dieses Abtragen von infiziertem/nekrotischem Gewebe wird als Debridement bezeichnet. Das Debridieren kann durch Wundspülung mit sogenannten Lavage-Systemen sowie durch Ausschneiden, Raspeln, Sägen und auch Bürsten erfolgen. Die zum Debridieren verwendeten Vorrichtungen sind nach dem Debridement mit Geweberesten und mit mikrobiellen Keimen kontaminiert. Für eine neue Verwendung müssen diese Instrumente sorgfältig gereinigt und anschließend sterilisiert werden. Dabei muss sich das medizinische Personal vor einer Kontamination beziehungsweise einer Infektion durch Übertragung von mikrobiellen Keimen während der Reinigungsarbeiten schützen. Daher ist es wünschenswert, wenn eine kostengünstige Vorrichtung mit Motorantrieb zum Raspeln, Sägen und Bürsten sowie zum Lavagieren für septische Revisionen bereitgestellt werden könnte, die nach einmaliger Verwendung ohne aufwendige und nicht gefahrlose Reinigungsschritte mit dem üblichen OP-Abfall zusammen entsorgt werden könnte. Es wäre dann besonders aus Gründen des Ressourcen- und Umweltschutzes aber auch aus Kostengründen sinnvoll, wenn für den Antrieb keine Batterien, Akkumulatoren und Elektromotoren notwendig wären.

In der Medizin sind gegenwärtig mit Druckluft und mit elektrischer Energie betriebene Antriebsvorrichtungen bekannt. Die auf Druckluft basierenden Antriebssysteme enthalten meisten Lamellenmotoren. Problematisch ist, dass unsterile Druckluft mit einem Schlauch zugeführt und nach dem Antrieb des Motors die entspannte, unsterile Luft in einem separaten Schlauch wieder aus dem Operationssaal abgeführt werden muss. Vielfach werden dazu koaxiale Schlauchsystem eingesetzt. Die mit Druckluft betriebenen Antriebe wurden weitgehend durch elektrisch betriebene Antriebssysteme ersetzt. Diese Antriebssysteme enthalten Elektromotoren mit Getrieben und nutzen als Energiequelle üblicherweise Akkumulatoren. Die elektrisch betriebenen Antriebssysteme enthalten Kupfer und weitere Schwermetalle.

Bei Lavage-Systemen werden Sprühstrahlen aus Spülflüssigkeiten erzeugt, die auf die zu reinigenden Gewebeareale auftreffen und eine mechanische Reinigungswirkung auf diese Gewebeareale ausüben. Gepulste Lavage-Systeme sind seit langem bekannt, beispielsweise aus der US 4 583 531 A, der US 4 278 078 A und der US 5 542 918 A.

Mit dem Patent US 9 861 770 B1 wird ein Vakuummotor vorgeschlagen, bei dem durch Einwirken von Vakuum auf einen Kolben eine Spiralfeder gespannt wird. Am Ende des Spannvorgangs wird ein Impuls auf einen Ventilkolben erteilt, der Belüftungsöffnungen freigibt und kurzzeitig die Vakuumöffnungen verschließt. Die gespannte Spiralfeder kann sich entspannen und treibt den Kolben in die Ausgangslage zurück und treibt damit einen verbundenen Pumpkolben an. Es entsteht eine periodische, lineare, oszillierende Pumpbewegung, die von der Spiralfeder angetrieben wird, wobei das Spannen der Spiralfeder durch eine Druckdifferenz erfolgt. Nachteilig ist hieran, dass der Vakuummotor viele Teile und vor allem mindestens drei gegeneinander bewegliche Teile plus die Spiralfeder aufweist. Bei allen innerhalb des Vakuummotors beweglichen Teilen besteht die Gefahr, dass diese blockieren oder bei hohen Frequenzen aufgrund von Reibung überhitzen. Ein noch einfacherer Vakuummotor wäre wünschenswert.

Aus der US 5 542 918 A ist eine Flüssigkeitspumpe für ein Lavage-System bekannt, das von einem Unterdruck angetrieben wird und bei dem eine Membran von einem hohlförmigen, linear beweglichen und federnd gelagerten Kolben angetrieben wird, in dem ein ebenfalls federnd gelagerter Ventilkolben zum Öffnen und Schließen eines Gaseinlasses zum Zuführen von Luft angeordnet ist. Das System hat den Nachteil, dass das Ventilelement im geöffneten Zustand nur einen geringen freien Strömungsquerschnitt bereitstellen kann. Dadurch ist die mit der Pumpe erzeugbare Kraft begrenzt und es besteht die Gefahr eines Totpunkts, aus dem heraus die Pumpe nicht von alleine wieder startet. Zudem kann es durch die fehlende Führung des Ventilkolbens zu seitlichen Bewegungen des Ventilkolbens und dadurch zu unregelmäßigen Schwingungen des Kolbens kommen.

Eine weitere Pumpe zum Erzeugen von gepulsten Flüssigkeitsstrahlen ist aus der DE 10 2011 018 708 A1 bekannt. Auch hier wirkt ein Kolbenvibrator auf eine Membran ein, die als Pumpe genutzt wird. Der Kolben des Kolbenvibrators ist aufwendig im Aufbau. Zudem müssen die Maße beim Aufbau sehr genau eingehalten werden, um einen störungsfreien Betrieb zu gewährleisten.

Die US 2005/0084395 A1 offenbart ein vakuumgetriebenes Lavage-System, das über zwei Zylinder arbeitet. In den Zylindern werden zwei miteinander gekoppelte Kolben von dem Vakuum angetrieben. Auch dieser Aufbau ist durch die miteinander über Gelenke gekoppelten Kolben sehr aufwendig in der Realisierung.

In dem Patent US 8 292 909 B1 wird ein doppeltwirkender Vakuummotor beschrieben. Bei diesem wird am Ende der Vorwärts- und der Rückwärtsbewegung des Arbeitskolbens eine Kraft auf einen Ventilkolben durch ein bistabiles Kopplungselement übertragen, wodurch dieser von einem Schaltzustand in einem zweiten Schaltzustand übergeht. Der Ventilkolben verharrt dadurch in einem definierten Schaltzustand während der Bewegung des Arbeitskolbens. Es wird somit durch die jeweilige Endposition des Arbeitskolbens der Schaltzustand des Ventilkolbens definiert. Auch dieses Konzept erfordert einen hohen Fertigungs- und Montageaufwand.

Für das Sprühen von Flüssigkeiten reichen die mit den Vakuummotoren erreichbaren Kräfte völlig aus. Für den Antrieb von Sägen, Raspeln und Bürsten sind jedoch größere Antriebskräfte notwendig.

Die Verwendung von Druckgasmotoren zum Antrieb von chirurgischen Werkzeugen ist bekannt. Dabei kommen neben Lamellenmotoren vor allem Druckluftturbinen zum Einsatz. Die entspannte Luft wird zumeist mit einer speziellen Leitung zurückgeführt, weil die eingesetzte Druckluft nicht steril ist. Die wiederverwendbaren chirurgischen Druckgasmotoren müssen nach jeder Anwendung gereinigt und sterilisiert oder zumindest desinfiziert werden. Bei septischen Operationen gibt es naturgemäß eine massive mikrobielle Kontamination der eingesetzten chirurgischen Instrumente und auch der Antriebsvorrichtungen. Die Reinigung und die Aufarbeitung sind aufwendig und gesundheitsgefährdend. Weiterhin ist eine Reinigungs- und Sterilisationsvalidierung bei medizinischen Antriebssystemen sehr kompliziert und mit hohen Kosten verbunden. Daher ist es wünschenswert, anstelle von resterilisierbaren chirurgischen Antriebsvorrichtungen einmalig zu verwendende Antriebsvorrichtungen zu entwickeln, die umweltfreundlich nach der Verwendung zusammen mit dem infektiösen Klinikabfall durch Verbrennung entsorgt werden können.

Bei der Verwendung von Druckluft-getriebenen Lavage-Systemen muss jedoch ein Doppelschlauchsystem verwendet werden, bei dem in einem Schlauch die unsterile Druckluft zugeführt wird und einen zweiten Schlauch mit dem die nach dem Antrieb des Druckluftmotors zumindest teilentspannte unsterile Luft abgeführt wird. Bei den mit Druckluft oder einem anderen komprimierten Gas getriebenen Systemen wird üblicherweise ein Druckgasmotor zum Antrieb eingesetzt.

Druckgasmotoren gehören zur Gruppe der Gasexpansionsmotoren. Das bedeutet, der Antrieb erfolgt durch eine Expansion (insbesondere durch eine adiabatische Expansion) eines unter Druck stehenden Gases. Die üblichen Gasexpansionsmotoren/Druckgasmotoren können unterteilt werden in Lamellenmotoren, Zahnradmotoren, Druckluftturbinen und Kolbenmotoren. Bei den bisher bekannten Kolbenmotoren nutzen Ein- und Auslassventile zur Steuerung des Einlasses von Druckgas und des Auslasses des entspannten Gases. Es werden zur Steuerung des Ein- und Auslasses anstelle von Ventilen auch Schieber verwendet. Die meisten bisherigen Druckgasmotoren werden im Wesentlichen aus Stahl gefertigt und sind für eine dauerhafte Verwendung vorgesehen.

Ein solcher Druckgasmotor ist aus der WO 2012/038003 A1 bekannt. Der dort beschriebene Druckgasmotor hat einen zweiteiligen Kolben mit einem Zwischenraum und einen Durchgang durch einen der Kolbenteile. Dadurch ist der Motor besonders einfach und kostengünstig im Aufbau. Ein Druckgasmotor für eine Sprühdose ist aus der DE 37 24 110 A1 bekannt. Eine mit Druckgas angetriebene Hubkolbenpumpe ist aus der US 4 993 924 A bekannt. Ein Druckgasmotor für ein Lavage-System ist aus der EP 2 873 856 A1 bekannt. Bei diesem Druckgasmotor wird ein Steuerkolben von einem Arbeitskolben über ein Mitnehmerelement bewegt und dadurch werden im Betrieb des Druckgasmotors eine Gaseinlassöffnung und eine Gasauslassöffnung periodisch geöffnet und geschlossen. Ein Druckgasmotor mit einem Kolbensystem ist ferner aus der DE 10 2010 046 057 B3 bekannt. Die WO 01/43651 A1 offenbart ein medizinisches Instrument zum Abtragen von Gewebe oder Knochenzement mit einem Druckgasmotor, bei dem ein abgedichteter Kolben angetrieben von Druckluft periodisch über eine Entlüftungsöffnung und gegen eine Rückstellfeder läuft.

In der DE 28 16 617 C2 wird ein Druckluftmotor mit einem doppeltwirkenden Kolben offenbart, bei dem die Druckluftzufuhr über separate Schieber gesteuert wird. Ähnlich arbeitende Druckluftmotoren wurden in den Dokumenten DE 24 28 853 C3, DE 1 476 673 A1, US 2 601 848 A und US 2 000 890 A beschrieben.

Es besteht immer der Wunsch nach einem kostengünstiger aufzubauenden Motor. Zudem besteht auch ein Bedürfnis danach, einen Motor bereitzustellen, der mit einer höheren Frequenz und/oder einer größeren Kraft betrieben werden kann.

Die US 5 554 011 A offenbart eine Pumpe mit einem Druckgasmotor, bei dem ein Ventilelement durch die Bewegung einer Membran gesteuert wird. An der Membran wird die Arbeit durch den wechselnden Gasdruck geleistet und dadurch die Pumpe angetrieben. Der Aufbau dieses Druckgasmotors ist relativ aufwendig und es kann aufgrund der Membran und der notwendigen Verspannung der Membran bei gleichartig aufgebauten Druckgasmotoren zu unterschiedlichem Schwingungsverhalten kommen. Zudem besteht bei dem Ventilelement die Gefahr eines Totpunkts, aus dem Heraus der Druckgasmotor nicht mehr selber weiterlaufen kann.

In den Patenten EP 2 910 270 B1 und US 9 861 770 B2 wird ein Druckgasmotor zum Antrieb einer Pumpe für ein Lavage-System beschrieben. Der Druckgasmotor besteht aus einem axial in einem Hohlraum beweglichen Arbeitskolben und einem dahinter angeordneten Rückstellelement. Der Arbeitskolben hat ein Mitnehmerelement, das im Betrieb einen Steuerkolben bewegt. Der Steuerkolben ist ein Hohlzylinder und kann über seine geschlossene Mantelfläche eine Gasauslassöffnung und eine Gaseinlassöffnung verdecken. An die Gasauslassöffnung ist eine Unterdruckquelle wie beispielsweise eine Vakuumpumpe angeschlossen. Das bedeutet, dass beim Betrieb des Motors periodisch die Gasauslassöffnung und die Gaseinlassöffnung verdeckt werden. Bei geöffneter Gasauslassöffnung wird die Luft aus dem Druckgasmotor gesaugt, wobei der Arbeitskolben gegen das Rückstellelement bewegt wird. Die Zuluftöffnung ist gleichzeitig durch den Steuerkolben verschlossen. Das Rückstellelement wird gespannt. Dann erteilt das Mitnehmerelement dem Steuerkolben einen Impuls. Der Steuerkolben bewegt sich vom Arbeitskolben weg und öffnet die Gaseinlassöffnung und verschließt gleichzeitig die Gasauslassöffnung. Das Vakuum bricht zusammen und das Rückstellelement bewegt den Arbeitskolben in seine Ausgangslage. Dabei erteilt der Mitnehmer dem Steuerkolben einen Impuls. Der Steuerkolben verschiebt sich in Richtung Arbeitskolben. Die Gaseinlassöffnung wird durch die Mantelfläche des Steuerkolbens verschlossen und die Gasauslassöffnung wird geöffnet. Dann beginnt der Zyklus erneut. Dieser Druckgasmotor ist für hohe Pulszahlen von ca. 2000 Pulsen pro Minute zum Betrieb einer Pumpe für ein Lavage-System ausgelegt. Die Hublänge ist relativ kurz und beträgt zwischen 2 und 5 mm. Für das Bürsten, Raspeln oder Sägen werden größere Hublängen und geringe Pulszahlen benötigt. Zudem ist auch dieser Aufbau aufwendig. Für die Fertigung des in den Patenten EP 2 910 270 B1 und US 9 861 770 B2 beschriebenen Druckgasmotors sind durch Kunststoffpräzisionsspritzgießen gefertigte Bauteile notwendig. Die Montage dieser Teile bedarf einer hohen Sorgfalt.

Ein weiterer Druckgasmotor für ein Lavage-System ist aus der EP 2 939 699 B1 bekannt. Bei diesem Druckgasmotor wird ein Vibrationskörper mit einem durch den Druckgasmotor geleiteten Druckgas in Schwingung versetzt, mit einem Kolben, der mit einem Rückstellelement federnd gelagert ist, wobei der Vibrationskörper und der Kolben derart positioniert und gelagert sind, dass der Vibrationskörper bei der Schwingung wiederholt auf den Kolben trifft und diesen gegen das Rückstellelement auslenkt. Nachteilig ist an dieser Vorrichtung, dass zwei gegeneinander bewegliche Teile in dem Druckgasmotor aufeinandertreffen und somit die Schwingung nicht beliebig gleichmäßig erfolgen muss, so dass der Druckgasmotor nicht mehr gut zum Antreiben von Sägen, Raspeln oder Bürsten verwendbar ist. Zudem ist es möglich, den Aufbau des Druckgasmotors immer noch zu vereinfachen und damit kostengünstiger zu gestalten.

Ein Druckgasmotor mit einseitig mit Druckluft zu beaufschlagendem Kolben und einer Rückholfeder wurde in der US 5 924 602 A vorgeschlagen. Dabei ist in dem Kolben ein Ventil angeordnet, das nach Erreichen einer Endposition den mit Druckluft beaufschlagtem Raum hinter dem Kolben öffnet und die zumindest teilentspannte Druckluft in die Umgebung entlässt. Der Ventilmechanismus ist sehr komplex und der Aufbau daher aufwendig.

Ein ähnlich wirkender Druckgasmotor wurde in den Schriften US 6 736 292 B2 und US 6 923 348 B2 beschrieben. Bei diesem Druckgasmotor ist ein Ventilkörper axial verschiebbar innerhalb eines Kolbens angeordnet. In einem Antriebszylinder ist eine umlaufende Nut angeordnet. Der Kolben hat an einer Mantelfläche zwei voneinander beabstandete umlaufende Dichtungen. Zwischen den Dichtungen ist eine gasdurchlässige Öffnung angeordnet, die mit dem axial beweglichen Ventilkörper gasdurchlässig verbunden ist. Beim Überfahren der umlaufenden Nut des Antriebszylinders durch die erste und zweite Dichtung des Kolbens strömt Druckluft durch die Nut unter der zweiten Dichtung in die seitliche Öffnung des Kolbens. Dadurch wird der Ventilkörper durch die einströmende Druckluft in Richtung des Bodens des Arbeitszylinders gedrückt. Das Ventil öffnet einen Kanal und die zumindest teilentspannte Druckluft kann durch diesen Kanal durch den Kolben in die umgebende Atmosphäre entweichen. Danach schiebt die Rückholfeder den Kolben über die Nut in den Ausgangszustand, wobei das Ventil in seine Ausgangslage zurückkehrt und der gesamte Prozess wiederholt sich solange Druckluft am Druckgasmotor anliegt. Das Überfahren der Nut mit den Dichtungen kann Probleme bereiten. Insbesondere kann es durch Erhitzen aufgrund von Reibung zu einer Veränderung der Arbeitsweise des Druckgasmotors kommen. Ferner ist die Herstellung und die Montage der Teile des Druckgasmotors aufwendig.

Die Aufgabe der vorliegenden Erfindung besteht also darin, die Nachteile des Stands der Technik zu überwinden. Insbesondere besteht die Aufgabe der Erfindung in der Entwicklung eines Druckgasmotors, der kostengünstig und einfach zu fertigen ist, der zuverlässig arbeitet, vielseitig einsetzbar ist und mit Hilfe von Druckluft, wie sie in Krankenhäusern zur Verfügung steht, oder mit Hilfe eines unter Druck stehenden Gases aus einer Druckgaspatrone eine ausreichende Leistung erzielt, um Gewebe zu debridieren und/oder Werkzeuge für die Chirurgie anzutreiben. Daher ist es auch Aufgabe der vorliegenden Erfindung, derartige chirurgische Werkzeuge und Lavage-Systeme mit einem solchen Druckgasmotor beziehungsweise ein chirurgisches Antriebssystem hiermit bereitzustellen. Ebenso ist es Aufgabe der vorliegenden Erfindung ein Verfahren zum Betreiben eines Druckgasmotors bereitzustellen, wobei das Verfahren die oben zum Druckgasmotor genannten Vorteile bieten soll.

Aufgabe der Erfindung ist es auch, einen extrem vereinfachten, kostengünstigen Druckgasmotor zu entwickeln, der mit Druckluft oder mit Druckgas angetrieben werden kann und der eine oszillierende, lineare Bewegung erzeugt. Der Druckgasmotor soll einfacher aufzubauen und kostengünstiger zu fertigen sein als bekannte Druckgasmotoren für den Antrieb eines Lavage-Systems oder für chirurgische Werkzeuge. Der Druckgasmotor soll zum Antreiben von Werkzeugen, wie Raspeln, Sägen oder Bürsten, zum Antreiben von Lavage-Systemen und von Vorrichtungen zum Debridement von infiziertem Weich- und Knochengewebe geeignet sein, die aus hygienischen Gründen nur zum einmaligen Gebrauch als sogenannte "disposable devices" (Wegwerfprodukte) bestimmt sind. Der Druckgasmotor soll mit Ethylenoxid sterilisierbar sein.

Die Aufgabe der Erfindung besteht auch darin, einen maximal vereinfachten Druckgasmotor zu entwickeln, der ein chirurgisches Antriebssystem antreiben soll. Das chirurgische Antriebssystem ist zur einmaligen Verwendung bestimmt. Der Druckgasmotor soll aus einer möglichst geringen Anzahl von Teilen bestehen und mit minimalem Herstellungsaufwand gefertigt werden können. Der zu entwickelnde Druckgasmotor soll möglichst keine separaten Ventile, keine Ventilsteuerungen und auch keine Schiebersteuerungen enthalten. Der Druckgasmotor soll im Wesentlichen aus Kunststoffspritzgussteilen bestehen und umweltverträglich entsorgt werden können. Die Verwendung von Batterien und Akkumulatoren sowie von Elektromotoren soll vermieden werden. Weiterhin soll der Druckgasmotor in der Lage sein, chirurgisch übliche Sägeblätter und ähnliche Werkzeuge anzureiben. Der Druckgasmotor soll mit beliebigen Druckgasen und insbesondere mit Druckluft angetrieben werden können. Der Start des Druckluftmotors soll ohne Hilfsvorrichtungen lageunabhängig und zuverlässig erfolgen.

Die der vorliegenden Erfindung zugrundeliegenden Aufgaben werden gelöst durch einen Druckgasmotor aufweisend
A) einen Hohlzylinder, der durch eine Mantelwand begrenzt ist,
B) einen rückseitigen Verschluss an einer rückseitigen Grundfläche des Hohlzylinders,
C) einen im Hohlzylinder axial beweglichen Kolben, der an seiner Vorderseite mit einer Antriebsstange verbunden ist, die aus dem Hohlzylinder herausragt, wobei der Kolben den Hohlzylinder in zwei Innenräume trennt, und zwar in
D) einen hinteren Innenraum des Hohlzylinders, der durch den Kolben, die Mantelwand des Hohlzylinders und den rückseitigen Verschluss begrenzt ist, und
E) einen vorderen Innenraum des Hohlzylinders, der durch den Kolben und die Mantelwand des Hohlzylinders und eine vorderseitige Grundfläche des Hohlzylinders begrenzt ist,
F) mindestens eine Druckgaseinmündung, die in den hinteren Innenraum des Hohlzylinders einmündet,
G) mindestens eine Belüftungsöffnung in der Mantelwand des Hohlzylinders, wodurch der hintere Innenraum des Hohlzylinders mit der umgebenden Atmosphäre während des Betriebs des Druckgasmotors gasdurchlässig verbunden oder verbindbar ist, wobei die mindestens eine Belüftungsöffnung vom axial beweglichen Kolben während des Betriebs des Druckgasmotors zum hinteren Innenraum hin durch die Bewegung des Kolbens periodisch freilegbar ist,
H) mindestens eine im vorderen Innenraum angeordnete Druckfeder, wobei sich die mindestens eine Druckfeder während des Betriebs des Druckgasmotors zumindest zeitweise an der vorderseitigen Grundfläche des Hohlzylinders abstützt, und/oder
I) mindestens eine im hinteren Innenraum angeordnete Zugfeder, wobei die mindestens eine Zugfeder mit dem Kolben und mit dem rückseitigen Verschluss des Hohlzylinders verbunden ist, wobei
J) die mindestens eine Druckfeder den Kolben so weit in Richtung des rückseitigen Verschlusses drückt und/oder die mindestens eine Zugfeder den Kolben so weit in Richtung des rückseitigen Verschlusses zieht, dass der hintere Innenraum des Hohlzylinders gegenüber der mindestens einen Belüftungsöffnung vom Kolben verschlossen ist und der hintere Innenraum mit der Druckgaseinmündung verbunden ist, wenn im vorderen Innenraum des Hohlzylinders und im hinteren Innenraum des Hohlzylinders der gleiche Druck herrscht, und wobei zwischen dem Kolben und der Mantelwand des Hohlzylinders ein gasdurchlässiger Spalt vorhanden ist, durch den der hintere Innenraum und der vordere Innenraum gasdurchlässig miteinander verbunden sind.

Die mindestens eine Druckfeder und/oder die mindestens eine Zugfeder kann mit jeweils mindestens einem Federelement realisiert werden. Bevorzugt ist die mindestens eine Druckfeder und/oder die mindestens eine Zugfeder eine Spiralfeder. Es kann jedoch auch vorgesehen sein, dass die Feder eine Gasfeder, eine Schneckenfeder oder eine andere für den Zweck geeignete Feder ist. Stahlfedern sind dabei besonders bevorzugt.

Die mindestens eine Belüftungsöffnung verbindet vorzugsweise den vorderen Innenraum mit der Umgebung des Druckgasmotors, wenn die mindestens eine Druckfeder und/oder die mindestens eine Zugfeder den Kolben bei nicht mit Gasdruck beaufschlagtem hinteren Innenraum vollständig in Richtung des rückseitigen Verschlusses ausgelenkt hat oder haben.

Ein Zylinder beziehungsweise eine zylindrische Geometrie im Sinne der vorliegenden Erfindung ist, wie auch nach der allgemeinen Definition, ein von zwei parallelen, ebenen, kongruenten Flächen (die rückseitige Grundfläche und die vorderseitige Grundfläche) und einer Mantelfläche beziehungsweise Zylindermantelfläche begrenzter Körper, wobei die Mantelfläche von parallelen Geraden gebildet wird. Das heißt, der Zylinder entsteht durch Verschiebung einer ebenen Fläche entlang einer Geraden, die nicht in dieser Ebene liegt. Die Höhe und die Achse des Zylinders ist gegeben durch den Abstand der beiden Ebenen, in denen die rückseitige Grundfläche und die vorderseitige Grundfläche liegen.

Sind die Geraden senkrecht zu den Grundflächen, spricht man von einem geraden Zylinder. Die gerade zylindrische Geometrie des Hohlzylinders ist erfindungsgemäß bevorzugt. Ein gerader Kreiszylinder stellt im Sinne der vorliegenden Erfindung also nur einen Spezialfall einer zylindrischen Geometrie dar, ist aufgrund der einfacheren Fertigung aber ebenfalls bevorzugt.

Vorliegend wird unter einer axialen Richtung die Richtung bezogen auf die Zylinderachse des Hohlzylinders verstanden.

Bevorzugt ist der axial bewegliche Kolben ein rotationssymmetrischer Körper, der entlang seiner Symmetrieachse beweglich in dem Hohlzylinder angeordnet ist.

Es kann bevorzugt vorgesehen sein, dass der Hohlzylinder durch zwei geschlossene Grundflächen begrenzt ist. Dabei kann vorgesehen sein, dass die Antriebsstange durch eine oder zwei Aussparungen in der vorderseitigen Grundfläche aus dem Hohlzylinder herausragt. Die Antriebsstange kann dazu durch einen vorderseitigen Verschluss des Hohlzylinders hindurchgeführt sein.

Die Mantelwand ist bevorzugt die Wandung, die den Hohlzylinder an seinem Zylindermantel begrenzt. Unter dem Zylindermantel ist die Fläche zu verstehen, die parallel zu der Zylinderachse des Hohlzylinders und umlaufend um die Zylinderachse des Hohlzylinders verläuft.

Die vorderseitige Grundfläche des Hohlzylinders ist der rückseitigen Grundfläche des Hohlzylinders gegenüberliegend angeordnet.

Es ist vorgesehen, dass der Kolben nicht mit einer Dichtung gegen den Hohlzylinder abgedichtet ist. Hierdurch wird die Reibung zwischen dem Kolben und dem Hohlzylinder reduziert und ein kühlender Luftstrom erzeugt, auf dem der Kolben gleiten kann.

Auch ist vorgesehen, dass der Kolben nicht passgenau in dem Hohlzylinder steckt und/oder dass der Kolben gegen den Hohlzylinder Spiel hat, bevorzugt zumindest 0,5 µm Spiel hat, besonders bevorzugt zumindest 1 µm Spiel hat, ganz besonders bevorzugt zwischen 1 µm und 10 µm Siel hat.

Bevorzugt kann vorgesehen sein, dass die mindestens eine Druckgaseinmündung in der Mantelwand der Hohlzylinders und/oder in dem rückseitigen Verschluss angeordnet ist, wobei besonders bevorzugt die mindestens eine Druckgaseinmündung in der Mantelwand des Hohlzylinders angeordnet ist. Es wird erfindungsgemäß bevorzugt, wenn die mindestens eine Druckgaseinmündung eine einzige Druckgaseinmündung ist.

Die Zugfeder gilt im Rahmen der vorliegenden Erfindung auch dann mit dem rückseitigen Verschluss des Hohlzylinders als verbunden, wenn Sie mit der an den rückseitigen Verschluss angrenzenden Mantelwand des Hohlzylinders im Bereich des hinteren Innenraums des Hohlzylinders verbunden ist.

Die Vorderseite des Druckgasmotors ist die Seite, an der die Antriebsstange aus dem Hohlzylinder austritt. Mit der Antriebsstange kann ein Werkzeug, wie beispielsweise eine Säge, eine Knochensäge, eine Raspel oder eine Bürste angetrieben werden. Auch kann mit der Antriebsstange eine Membran einer Membran-Pumpe bewegt beziehungsweise angetrieben werden.

Die Arbeitsfläche des Kolbens, an der die Arbeit von dem Druckgas verrichtet wird, ist die Rückseite des Kolbens, die den hinteren Innenraum des Holzylinders an seiner Vorderseite begrenzt. Dementsprechend kann der hintere Innenraum des Hohlzylinders als der Arbeitsraum des Druckgasmotors aufgefasst werden.

Unter dem Begriff "Druckgas" werden alle unter Druck stehenden Gase verstanden, die einen höheren Druck als die umgebende Atmosphäre haben. Bevorzugt wird als Druckgas Druckluft mit Drücken größer 3 bar oder Gas aus einer CO₂-Patrone verwendet.

Bei erfindungsgemäßen Druckgasmotoren kann vorgesehen sein, dass der Druckgasmotor einen vorderseitigen Verschluss der vorderseitigen Grundfläche des Hohlzylinders aufweist, wobei der vorderseitige Verschluss eine Durchbrechung aufweist, insbesondere eine zentrale axiale Durchbrechung aufweist, durch die die Antriebsstange hindurch geführt ist und wobei der vordere Innenraum des Hohlzylinders durch den Kolben, die Innenwand des Hohlzylinders und den vorderseitigen Verschluss begrenzt ist, wobei bevorzugt der vorderseitige Verschluss zumindest eine gasdurchlässige Öffnung aufweist.

Hierdurch wird der Hohlzylinder nach außen abgeschlossen. Nur die durch die Lagerung im vorderseitigen Verschluss stabilisierte Bewegung der Antriebsstange kann von außen beeinträchtigt beziehungsweise genutzt werden. Eine Blockade des Kolbens kann somit verhindert werden oder die Wahrscheinlichkeit hierfür reduziert werden.

Der vorderseitige Verschluss enthält bevorzugt zumindest eine gasdurchlässige Öffnung. Dadurch kann Luft bei der Bewegung des Kolbens in Richtung des vorderseitigen Verschlusses aus dem vorderen Innenraum in die umgebende Atmosphäre austreten. Ein Überdruck im vorderen Innenraum, der zu einer Bremswirkung führen würde, wird dadurch verhindert. Zudem kann ein Gasstrom beziehungsweise Gasfilm zwischen dem hinteren Innenraum und dem vorderen Innenraum durch einen Spalt zwischen dem Kolben und der Mantelwand aufrechterhalten werden, auf dem der Kolben gleiten kann und der die Reibungswärme abführt. Bei der Rückbewegung des Kolbens in Richtung des rückseitigen Verschlusses wird durch die Kolbenbewegung Luft in den vorderen Innenraum gesaugt. Wenn keine oder zu kleine gasdurchlässige Öffnungen im vorderseitigen Verschluss vorhanden sind, wird das Einströmen der Luft in den vorderen Innenraum gebremst oder verhindert. Dadurch kann es zu einer Bremsung der Bewegung des Kolbens und der Antriebsstange kommen. Es ist auch zusätzlich oder alternativ möglich, zumindest eine gasdurchlässige Öffnung unmittelbar vor dem vorderseitigen Verschluss in der Mantelwand des Hohlzylinders anzuordnen.

Des Weiteren kann vorgesehen sein, dass der Kolben den Hohlzylinder in zwei voneinander separate Innenräume, nämlich den vorderen Innenraum und den hinteren Innenraum, trennt, wobei der Kolben den Hohlzylinder nicht gasdicht in zwei voneinander separate Innenräume trennt.

Hiermit wird sichergestellt, dass durch einen Druckaufbau im hinteren Innenraum der Kolben in Richtung des vorderen Innenraums beschleunigt werden kann.

Es kann erfindungsgemäß vorgesehen sein, dass eine Druckgasleitung mit der Druckgaseinmündung verbunden ist, so dass die Druckgasleitung mit dem hinteren Innenraum des Hohlzylinders gasdurchlässig verbunden ist.

Hiermit wird das Einspeisen von Druckgas durch die Druckgasleitung in den hinteren Innenraum vereinfacht.

Ferner kann vorgesehen sein, dass die Antriebsstange parallel zur Zylinderachse des Hohlzylinders angeordnet ist, bevorzugt auf der Zylinderachse des Hohlzylinders angeordnet ist.

Hiermit wird die Bewegung der Antriebsstange passend zur Richtung der durch den Gasdruck wirkenden Kraft ausgerichtet. Dadurch können ein Verklemmen und eine Blockade des Kolbens sowie Energieverluste bei der Bewegung des Kolbens im Hohlzylinder vermieden werden.

Zu dem gleichen Zweck kann vorgesehen sein, dass der Kolben und die Antriebsstange linear beweglich, insbesondere axial beweglich bezogen auf eine Symmetrieachse des Kolbens und/oder der Antriebsstange, in dem Hohlzylinder angeordnet sind.

Ferner kann vorgesehen sein, dass bei Einspeisen eines unter Druck stehenden Druckgases durch die mindestens eine Druckgaseinmündung in den hinteren Innenraum des Hohlzylinders der Kolben durch periodische Wechsel der Einwirkung des Druckgases und der umgebenden Atmosphäre im hinteren Innenraum eine oszillierende Bewegung der Antriebsstange bewirkt, wobei bevorzugt die mindestens eine Druckfeder und/oder die mindestens eine Zugfeder durch die Bewegung des Kolbens in Richtung der vorderen Grundfläche des Hohlzylinders angetrieben von dem Druckgas zu spannen ist und der Kolben von der gespannten mindestens einen Druckfeder und/oder der gespannten mindestens einen Zugfeder in Richtung der rückseitigen Grundfläche zu drücken ist, wenn der hintere Innenraum zur umgebenden Atmosphäre geöffnet ist.

Hiermit wird sichergestellt, dass der Druckgasmotor konstant läuft, während das Druckgas den Druckgasmotor beziehungsweise den hinteren Innenraum durchströmt.

Gemäß einer bevorzugten Weiterbildung der Kolben einen Durchmesser von größer oder gleich 20 mm hat, bevorzugt von größer oder gleich 30 mm und besonders bevorzugt von größer oder gleich 35 mm hat.

Der Durchmesser bezieht sich dabei auf die Abmessung des (vorzugsweise zumindest bereichsweise zylindrischen) Kolbens senkrecht zur Zylinderachse des Hohlzylinders.

Durch diese Maßnahmen wird erreicht, dass mit einem üblichen Druckgas oder in Krankenhäusern vorhandener Druckluft eine für medizinische Werkzeuge ausreichende Kraft als Antrieb zur Verfügung gestellt werden kann.

Auch kann vorgesehen sein, dass an der Vorderseite der zumindest einen Antriebsstange ein Befestigungselement, insbesondere ein Gewinde, angeordnet ist, über das ein Werkzeug, wie eine Säge, eine Raspel oder eine Bürste, mit einem zum Befestigungselement passenden Gegenbefestigungselement, insbesondere einem zum Gewinde passenden Gegengewinde, an der zumindest einen Antriebsstange befestigbar ist.

Hierdurch kann der Druckgasmotor zum Antreiben von unterschiedlichen Werkzeugen verwendet werden, die an dem Befestigungselement der Antriebsstange befestigt werden können.

Es kann erfindungsgemäß vorgesehen sein, dass die Antriebsstange mit einem Befestigungselement für ein Werkzeug ausgerüstet ist. Als Werkzeuge können Sägeblätter, Raspeln, Bürsten und Lavage-Aufsätze, die als pulsierende Pumpe arbeiten, angeschlossen werden. Weiterhin ist es möglich, die Antriebsstange mit einem Getriebe zu koppeln, das die lineare oszillierende Bewegung der Antriebsstange in eine Drehbewegung umformt.

Bevorzugt kann ferner vorgesehen sein, dass die den Hohlzylinder begrenzende Mantelwand, der Kolben, der rückseitige Verschluss und, sofern vorhanden auch ein vorderseitiger Verschluss, aus einem Kunststoff gefertigt sind, insbesondere aus einem thermoplastischen Kunststoff gefertigt sind, vorzugsweise durch Spritzgießen geformt sind.

Hierdurch kann der Druckgasmotor als kostengünstiges und hygienisch zu entsorgendes Einmalwerkzeug bereitgestellt werden.

Des Weiteren kann vorgesehen sein, dass die mindestens eine Druckfeder eine Spiralfeder ist, wobei die Spiralfeder die Antriebsstange umschließt, wobei vorzugsweise keine Zugfeder in dem hinteren Innenraum angeordnet ist.

Hierdurch wird der Druckgasmotor besonders einfach aufgebaut. Gleichzeitig kann auch eine zuverlässige Funktion des Druckgasmotors bereitgestellt werden. Durch die Anordnung der Spiralfeder um die Antriebsstange herum ist die Spiralfeder immer geführt und kann nicht aus der Längsachse ausweichen. Die Spiralfeder ist vorzugsweise nicht am vorderseitigen Verschluss und/oder am Kolben fixiert. Die Spiralfeder kann alternativ auch im Bereich der vorderseitigen Grundfläche oder am vorderseitigen Verschluss und am Kolben fixiert sein, wodurch die Funktionsfähigkeit des Druckgasmotors jedoch nicht beeinträchtigt und auch nicht verbessert wird.

Erfindungsgemäß ist vorgesehen, dass zwischen dem Kolben und der Mantelwand des Hohlzylinders ein gasdurchlässiger Spalt vorhanden ist, durch den der hintere Innenraum und der vordere Innenraum gasdurchlässig miteinander verbunden sind, wobei bevorzugt der Spalt eine Querschnittsfläche aufweist, die weniger als 1 % der Querschnittsfläche des Hohlzylinders senkrecht zur Zylinderachse beträgt, besonders bevorzugt weniger als 0,5 %, ganz besonders bevorzugt weniger als 0,1 % der Querschnittsfläche des Hohlzylinders senkrecht zur Zylinderachse beträgt.

Es wurde überraschend gefunden, dass ein kleiner Spalt zwischen dem Kolben und der Mantelwand des Hohlzylinders dazu führt, dass der Kolben auf einem Gasfilm des strömenden Druckgases (als einer Art Luftkissen) gleiten kann und so der reibungsarmen Beweglichkeit des Kolbens im Hohlzylinder zuträglich ist. Es wird damit also eine Reibung des sich in dem Hohlzylinder bewegenden Kolbens an der Mantelwandung verhindert. Der Spalt darf dabei nicht zu groß sein, damit sich ein ausreichender Gasdruck im hinteren Innenraum aufbauen kann, um den Kolben zu beschleunigen. Der Gasfilm verhindert auch ein Überhitzen des Druckgasmotors während des Betriebs durch eine Verminderung der Reibung zwischen dem Kolben und der Mantelwand und durch eine Kühlung durch den expandierten Gasstrom des Druckgases.

Die Querschnittsfläche ist bezogen auf eine Achse senkrecht zur Zylinderachse des Hohlzylinders.

Vorzugsweise kann auch vorgesehen sein, dass der Spalt weniger als 50 µm breit ist, besonders bevorzugt weniger als 10 µm breit ist. Ferner kann vorgesehen sein, dass der Spalt mindestens 0,5 µm breit ist, besonders bevorzugt mindestens 1 µm breit ist.

Dieser Spalt kann wenige Mikrometer betragen. Kolbenringe oder O-Ringe sind für den Betrieb des Druckgasmotors nicht notwendig. Dadurch können Material- und Montagekosten niedrig gehalten werden. Der Vorteil des Spalts besteht darin, dass bei der Druckbeaufschlagung durch den Spalt strömendes Druckgas die Gleitreibung zwischen dem Kolbenrand und der Innenwand des Hohlzylinders senkt. Ein weiterer Vorteil ist, dass durch das strömende Druckgas die Reibungswärme zu mindestens teilweise abführt wird. Das Druckgas hat dadurch eine gewisse Kühlfunktion.

Des Weiteren kann vorgesehen sein, dass die Lage der mindestens einen Belüftungsöffnung in der Mantelwand entlang der axialen Erstreckung des Hohlzylinders den Hub des Kolbens mit der Antriebsstange bestimmt.

Hierdurch wird erreicht, dass die Rückstellung des Kolbens mit der mindestens einen Druckfeder und/oder der mindestens einen Zugfeder durch die Belüftung des hinteren Innenraums ausgelöst werden kann. Aufgrund der Massenträgheit des Kolbens und aufgrund der zum Druckausgleich notwendigen Zeit kommt es dabei zu einem Überschwingen über die mindestens eine Belüftungsöffnung hinaus.

Die Lage der mindestens einen Belüftungsöffnung entlang der axialen Erstreckung des Hohlzylinders definiert den Hub des Kolbens mit der Antriebsstange. Das bedeutet, je näher die mindestens eine Belüftungsöffnung in Richtung der vorderseitigen Grundfläche angeordnet ist, umso größer ist der Hub des Kolbens und der Antriebsstange. Je näher die mindestens eine Belüftungsöffnung in Richtung des rückseitigen Verschlusses angeordnet ist, umso geringer wird der Hub des Kolbens mit der Antriebsstange. Hierdurch kann der Hub des Kolbens und der Antriebsstange und damit auch die Frequenz des Druckgasmotors an die jeweilig gewünschte Anwendung des Druckgasmotors angepasst werden.

Bevorzugt kann vorgesehen sein, dass der Kolben im Betrieb des Druckgasmotors die mindestens eine Belüftungsöffnung vollständig überfährt oder wenigstens eine der mindestens einen Belüftungsöffnung vollständig überfährt.

Die der vorliegenden Erfindung zugrundeliegenden Aufgaben werden auch gelöst durch ein chirurgisches Antriebssystem aufweisend einen erfindungsgemäßen Druckgasmotor sowie ein Ventilelement, insbesondere ein manuell bedienbares Ventilelement, wobei das Ventilelement in einer Druckgasleitung angeordnet ist, die mit einer der mindestens einen Druckgaseinmündung verbunden ist und die mit einem Druckgasreservoir verbindbar oder verbunden ist, so dass mit dem Ventilelement die Verbindung zu dem Druckgasreservoir unterbrechbar und/oder der Druck an der mindestens einen Druckgaseinmündung einstellbar ist.

Das chirurgische Antriebssystem weist die Vorteile des erfindungsgemäßen Druckgasmotors auf. Zudem ist es durch das bedienbare Ventilelement bequem einsetzbar.

Dabei kann vorgesehen sein, dass das chirurgische Antriebssystem einen Griff aufweist, mit dem es in einer Hand haltbar ist und das Ventilelement mit einem Abzug am Griff mit der gleichen Hand bedienbar ist.

Hierdurch wird die Handhabung des chirurgischen Antriebssystems vereinfacht.

Des Weiteren kann vorgesehen sein, dass in der Druckgasleitung ein Sterilfilter angeordnet ist.

Hiermit wird verhindert, dass bei der Anwendung des chirurgischen Antriebssystems kontaminierte oder nicht sterile Luft oder nicht steriles Druckgas in den OP-Saal gelangt. Der Sterilfilter filtriert das Druckgas und gewährleistet dadurch, dass das Druckgas keimfrei ist. Nach der Expansion im Druckgasmotor kann das entspannte Gas in die umgebende Atmosphäre abgegeben werden, ohne dass es mikrobielle Kontaminationen verursachen kann. Eine separate Leitung zum Rückführen des entspannten Druckgases ist dadurch nicht notwendig.

Bevorzugt kann vorgesehen sein, dass der Sterilfilter mit einem Überdruckventil verbunden ist. Dadurch wird eine Beschädigung des chirurgischen Antriebssystems durch einen eventuellen Überdruck des Druckgases im Sterilfilter verhindert.

Vorzugsweise weist das chirurgische Antriebssystem ein Gehäuse auf. Bevorzugt wird der Griff (sofern vorhanden) durch das Gehäuse ausgeformt. Als Gehäuse kommen übliche, durch Kunststoffspritzgießen hergestellte Kunststoffhalbschalen in Frage, die miteinander verrastet oder durch Schweißen, Vernieten, Verschrauben und/oder Verkleben miteinander verbunden werden können.

Die der vorliegenden Erfindung zugrundeliegenden Aufgaben werden auch gelöst durch ein medizinisches Lavage-System zum Debridement von Weichgewebe und/oder Knochengewebe aufweisend einen erfindungsgemäßen Druckgasmotor oder ein erfindungsgemäßes chirurgisches Antriebssystem.

Die der vorliegenden Erfindung zugrundeliegenden Aufgaben werden ferner auch gelöst durch eine medizinische Vorrichtung zum Bürsten, Raspeln oder Sägen von Weichgewebe und/oder Knochengewebe aufweisend einen erfindungsgemäßen Druckgasmotor oder ein erfindungsgemäßes chirurgisches Antriebssystem, wobei vorzugsweise an der Antriebsstange der medizinischen Vorrichtung ein Werkzeug, insbesondere eine Säge, eine Raspel oder eine Bürste befestigt ist.

Das medizinische Lavage-System oder die medizinische Vorrichtung weisen die Vorteile des erfindungsgemäßen Druckgasmotors oder des erfindungsgemäßen chirurgischen Antriebssystems auf.

Die der vorliegenden Erfindung zugrundeliegenden Aufgaben bezüglich des Verfahrens werden gelöst durch ein Verfahren zum Betreiben eines Druckgasmotors, bei dem ein Kolben linear und axial in einem Hohlzylinder oszilliert, wobei der Kolben mit einer Antriebsstange verbunden ist, die aus dem Hohlzylinder herausragt und der Kolben die Antriebsstange antreibt, wobei das Verfahren die folgenden chronologischen Schritte umfasst:
A) der Kolben begrenzt in einem Ausgangszustand einen hinteren Innenraum des Hohlzylinders, der bis auf mindestens eine Druckgaseinmündung gegenüber der Umgebung des Druckgasmotors geschlossen ist;
B) Einleiten eines Druckgases in den hinteren Innenraum des Hohlzylinders durch die mindestens eine Druckgaseinmündung;
C) Drücken des Kolbens mit der Antriebsstange in Richtung einer Vorderseite des Hohlzylinders unter Vergrößerung des hinteren Innenraums durch den Gasdruck des Druckgases im hinteren Innenraum;
D) Spannen mindestens einer Druckfeder und/oder mindestens einer Zugfeder durch die Bewegung der Kolbens;
E) Freilegen mindestens einer Belüftungsöffnung in einer Mantelwand des Hohlzylinders zum hinteren Innenraum durch die Bewegung des Kolbens, wobei die mindestens eine Belüftungsöffnung direkt in den hinteren Innenraum geöffnet wird;
F) Ausströmen des Druckgases aus dem hinteren Innenraum durch die mindestens eine Belüftungsöffnung,
G) Rückstellen des Kolbens durch eine Krafteinwirkung der mindestens einen Druckfeder und/oder mindestens einen Zugfeder auf den Kolben; und
H) Verschließen der mindestens einen Belüftungsöffnung gegen den hinteren Innenraum durch die Bewegung des Kolbens, wobei der Kolben in dem Hohlzylinder auf einem Gasfilm des Druckgases gleitet, der durch einen Spalt zwischen dem Kolben und der Innenwand des Hohlzylinders strömt.

Dabei kann vorgesehen sein, dass das Verfahren mit einem erfindungsgemäßen Druckgasmotor oder mit einem erfindungsgemäßen chirurgischen Antriebssystem oder mit einem erfindungsgemäßen Lavage-System oder einer erfindungsgemäßen medizinischen Vorrichtung zum Bürsten, Raspeln oder Sägen von Weichgewebe und/oder Knochengewebe durchgeführt wird.

Hierdurch ergeben sich die aus den konstruktiven Merkmalen ergebenden Fortschritte für den Ablauf des Verfahrens.

Bevorzugt kann vorgesehen sein, dass nach Schritt H) eine Wiederholung der Schritte B) bis H) oder C) bis H) erfolgt, solange über die mindestens eine Druckgaseinmündung ein Druckgas in den hinteren Innenraum eingespeist wird.

Hierdurch wird die Bewegung des Verfahrens periodisch fortgesetzt. Dadurch kann das Verfahren gut als Antrieb für ein Werkzeug oder eine Pumpe verwendet werden, solange Druckgas zur Verfügung steht.

Des Weiteren kann vorgesehen sein, dass mit der Bewegung der Antriebsstange beziehungsweise des Kolbens ein Werkzeug oder eine Pumpe angetrieben wird, insbesondere ein medizinisches Werkzeug, wie eine Säge, eine Raspel oder eine Bürste, oder ein Lavage-Aufsatz angetrieben wird.

Damit kann das Verfahren zum Pumpen, Sprühen, Sägen, Raspeln oder Bürsten verwendet werden, insbesondere im medizinischen Bereich.

Es ist vorgesehen, dass der Kolben in dem Hohlzylinder auf einem Gasfilm des Druckgases gleitet, der durch einen Spalt zwischen dem Kolben und der Innenwand des Hohlzylinders strömt.

Hiermit wird die Bewegung des Kolbens reibungsarm geführt, so dass ein größerer Teil der aufgebrachten Energie in die Bewegung umgesetzt wird und der Druckgasmotor im Betrieb nicht überhitzt. Insbesondere kann vorgesehen sein, dass der Kolben und die Mantelwand durch eine Strömung des in dem hinteren Innenraum entspannten Druckgases durch den Spalt gekühlt werden.

Der Erfindung liegt die überraschende Erkenntnis zugrunde, dass ein derart einfacher Aufbau mit nur einem Kolben, der gleichzeitig zum Öffnen und Schließen der mindestens eine Belüftungsöffnung und als Arbeitskolben verwendet wird, in einem Hohlzylinder einen zuverlässig arbeitenden Druckgasmotor, der vollständig aus Kunststoff oder weitgehend aus Kunststoff gefertigt werden kann, und gleichzeitig ein einfaches Verfahren zum Betrieb desselben ermöglicht. Dadurch ist der Druckgasmotor als kostengünstiger Wegwerfmotor in hygienisch sensiblen medizinischen Bereichen einsetzbar. Langlebigkeit ist dabei nicht erforderlich.

Die Erfindung basiert auch auf der überraschenden Erkenntnis, dass mit dem erfindungsgemäßen Druckgasmotor keine Ein- und Auslassventile oder Schieber notwendig sind, um einen Kolbenmotor mit Druckluft oder Druckgas anzutreiben, der eine periodische, linear oszillierende Bewegung erzeugt. Beim Einleiten von Druckgas in den hinteren Innenraum wird durch das expandierende Druckgas der Kolben mit der Antriebsstange im Bruchteil einer Sekunde in Richtung der vorderseitigen Grundfläche des Hohlzylinders bewegt, wobei gleichzeitig die mindestens eine Druckfeder komprimiert wird und/oder die mindestens eine Zugfeder gedehnt wird. Durch die Trägheit des Kolbens und insbesondere der Antriebsstange überfährt der Kolben die mindestens eine Belüftungsöffnung und komprimiert die mindestens eine Druckfeder und/oder dehnt die mindestens eine Zugfeder weiter. Das im hinteren Innenraum befindliche zumindest teilweise entspannte Gas kann durch die freigelegte mindestens eine Belüftungsöffnung in die umgebende Atmosphäre austreten. Der Druck im hinteren Innenraum nimmt ab. Gleichzeitig entspannt sich die mindestens eine Druckfeder und/oder die mindestens eine Zugfeder und schiebt beziehungsweise zieht den Kolben über die mindestens eine Belüftungsöffnung zurück in die Ausgangstellung. Durch das nachströmende Druckgas wiederholt sich der dargestellte Prozess.

Eigene Messungen zeigten, dass bis zu 4000 Hübe pro Minute mit einem solchen Druckgasmotor mit Druckluft mit einem Druck von 5 bar erreicht werden. Der Druckgasmotor startete dabei immer unabhängig davon, in welcher Lage er positioniert wurde. Überraschend ist, dass es mit dem aus Kunststoffen gefertigten Druckgasmotor möglich ist, bei Verwendung zumindest einer ausreichend starken Druckfeder und/oder Zugfeder, Sägen und Raspeln anzutreiben, für deren Antrieb eine hohe mechanische Kraft notwendig ist.

Ein beispielhafter erfindungsgemäßer Druckgasmotor ist zusammengesetzt aus
a) einem Hohlzylinder,
b) einem gasdichten rückseitigen Verschluss der rückseitigen Grundfläche des Hohlzylinders,
c) einem vorderseitigen Verschluss der vorderseitigen Grundfläche des Hohlzylinders, wobei der vorderseitige Verschluss eine zentrale Durchbrechung hat,
d) einem im Hohlzylinder axial beweglichen Kolben, der mit einer Stange als Antriebsstange verbunden ist, die aus der Durchbrechung des vorderseitigen Verschlusses herausragt,
e) einem hinteren Innenraum des Hohlzylinders, der durch den Kolben, der Innenwand des Hohlzylinders und den rückseitigen Verschluss begrenzt ist,
f) einem vorderen Innenraum des Hohlzylinders, der durch den Kolben, der Innenwand des Hohlzylinders und den vorderseitigen Verschluss begrenzt ist,
g) einer Druckgaszuleitung, die mit dem hinteren Innenraum gasdurchlässig verbunden ist,
h) mindestens einer Belüftungsöffnung im Mantel des Hohlzylinders, wodurch der vordere Innenraum des Hohlzylinders mit der umgebenden Atmosphäre gasdurchlässig verbindbar ist,
i) mindestens einer im vorderen Innenraum angeordneten Feder als Druckfeder, wobei sich die Feder am vorderseitigen Verschluss abstützt, und wobei
j) die Feder den Kolben im nicht mit Druckgas beaufschlagtem Zustand soweit in Richtung des rückseitigen Verschlusses schiebt, dass die mindestens eine Belüftungsöffnung den vorderen Innenraum belüftet und der hintere Innenraum gegenüber der umgebenden Atmosphäre verschlossen ist.

Der Hohlzylinder, der Kolben, der rückseitige Verschluss und (sofern vorhanden) der vorderseitige Verschluss können in einfacher Weise durch Kunststoffspritzgießen hergestellt werden. Die Antriebsstange sowie die mindestens eine Druckfeder und/oder die mindestens eine Zugfeder sind bevorzugt aus Metall, besonders bevorzugt aus Stahl gefertigt.

Eine beispielhafte medizinische Antriebsvorrichtung kann aufgebaut sein aus
1. einer flexiblen Druckgaszuführung,
2. einem mit der Druckgaszuführung gasdurchlässig verbundenen Sterilfilter,
3. einem mit dem Sterilfilter verbundenen und manuell zu bedienendem Ventil,
4. einer mit dem Ventil gasdurchlässig verbunden Druckgasleitung,
5. einem Druckgasmotor, der mit der Druckgasleitung gasdurchlässig verbunden ist, wobei der Druckgasmotor zusammengesetzt ist aus
   a) einem Hohlzylinder,
   b) einem gasdichten rückseitigen Verschluss der rückseitigen Grundfläche des Hohlzylinders,
   c) einem vorderseitigen Verschluss der vorderseitigen Grundfläche des Hohlzylinders, wobei der vorderseitige Verschluss eine zentrale Durchbrechung hat,
   d) einem im Hohlzylinder axial beweglichen Kolben, der mit einer Stange als Antriebsstange verbunden ist, die aus der Durchbrechung des vorderseitigen Verschlusses herausragt,
   e) einem hinteren Innenraum des Hohlzylinders, der durch den Kolben, der Innenwand des Hohlzylinders und den rückseitigen Verschluss begrenzt ist,
   f) einem vorderen Innenraum des Hohlzylinders, der durch den Kolben, der Innenwand des Hohlzylinders und den vorderseitigen Verschluss begrenzt ist,
   g) einer Druckgaszuleitung, die mit dem hinteren Innenraum gasdurchlässig verbunden ist,
   h) mindestens einer Belüftungsöffnung im Mantel des Hohlzylinders, wodurch der vordere Innenraum des Hohlzylinders mit der umgebenden Atmosphäre gasdurchlässig verbindbar ist,
   i) mindestens einer im vorderen Innenraum angeordneten Feder als Druckfeder, wobei sich die Feder am vorderseitigen Verschluss abstützt, und wobei
   j) die Feder den Kolben im nicht mit Druckgas beaufschlagtem Zustand soweit in Richtung des rückseitigen Verschlusses schiebt, dass die mindestens eine Belüftungsöffnung den vorderen Innenraum belüftet und der hintere Innenraum gegenüber der umgebenden Atmosphäre verschlossen ist,
6. einem Werkzeug, das mit der Stange des Druckgasmotors verbunden ist, und
7. einem Gehäuse, dass den Druckgasmotor, die Druckgasleitung, den Sterilfilter und das Ventil zu mindestens teilweise einhaust, wobei der Innenraum des Gehäuses gasdurchlässig mit der umgebenden Atmosphäre verbunden ist.

Der erfindungsgemäße Druckgasmotor kann als Antrieb von Lavage-Systemen und von Vorrichtungen zum Debridement von Weichgewebe und Knochengewebe verwendet werden. Der Druckgasmotor kann bevorzugt auch als Antrieb von Vorrichtungen zum Bürsten, Raspeln und Sägen von Weichgewebe und Knochengewebe verwendet werden. Weiterhin kann der Druckgasmotor zum Antrieb von einmalig zu verwendenden medizinischen Vorrichtungen verwendet werden.

Im Folgenden werden weitere Ausführungsbeispiele der Erfindung anhand von sechzehn schematisch dargestellten Figuren erläutert, ohne jedoch dabei die Erfindung zu beschränken. Dabei zeigt:
Figur 1: eine schematische Querschnittansicht eines beispielhaften erfindungsgemäßen Druckgasmotors im Ruhezustand;
Figur 2: eine schematische perspektivische Ansicht eines erfindungsgemäßen chirurgischen Antriebssystems enthaltend einen erfindungsgemäßen Druckgasmotor, wobei eine Knochensäge an dem Antriebssystem befestigt ist;
Figur 3: eine schematische Seitenansicht des chirurgischen Antriebssystems nach Figur 2;
Figur 4: eine schematische perspektivische Ansicht auf die Unterseite und Rückseite des chirurgischen Antriebssystems nach den Figuren 2 und 3;
Figur 5: eine schematische perspektivische Ansicht auf die Oberseite und Vorderseite des chirurgischen Antriebssystems nach den Figuren 2 bis 4 mit geöffnetem Gehäuse;
Figur 6: eine schematische perspektivische Querschnittssicht des chirurgischen Antriebssystems nach den Figuren 2 bis 5 mit dem Druckgasmotor im Ausgangszustand;
Figur 7: eine schematische Querschnittssicht des chirurgischen Antriebssystems nach den Figuren 2 bis 6 mit dem Druckgasmotor im Ausgangszustand;
Figur 8: eine schematische Querschnittssicht des chirurgischen Antriebssystems nach den Figuren 2 bis 7 mit dem Druckgasmotor im Ausgangszustand mit angelegtem Druckgas;
Figur 9: eine schematische Querschnittssicht des chirurgischen Antriebssystems nach den Figuren 2 bis 8 mit dem Druckgasmotor im Betrieb während des Einspeisens von Druckgas in den Druckgasmotor;
Figur 10: eine schematische Querschnittssicht des chirurgischen Antriebssystems nach den Figuren 2 bis 9 mit dem Druckgasmotor im Betrieb während des Einspeisens von Druckgas in den Druckgasmotor mit in Bewegung befindlichem Kolben;
Figur 11: eine schematische Querschnittssicht des chirurgischen Antriebssystems nach den Figuren 2 bis 10 mit dem Druckgasmotor im Betrieb während des Ablassens von Druckgas aus dem Druckgasmotor;
Figur 12: eine schematische perspektivische Ansicht eines erfindungsgemäßen chirurgischen Antriebssystems enthaltend einen erfindungsgemäßen Druckgasmotor, wobei ein Lavage-Aufsatz an dem Antriebssystem befestigt ist;
Figur 13: eine schematische perspektivische Querschnittansicht des chirurgischen Antriebssystems mit dem Lavage-Aufsatz nach Figur 12;
Figur 14: eine schematische perspektivische Ansicht eines erfindungsgemäßen chirurgischen Antriebssystems enthaltend einen erfindungsgemäßen Druckgasmotor, wobei eine Säge an dem Antriebssystem befestigt ist;
Figur 15: eine schematische perspektivische Ansicht eines erfindungsgemäßen chirurgischen Antriebssystems enthaltend einen erfindungsgemäßen Druckgasmotor, wobei eine Raspel an dem Antriebssystem befestigt ist; und
Figur 16: eine schematische perspektivische Ansicht eines erfindungsgemäßen chirurgischen Antriebssystems enthaltend einen erfindungsgemäßen Druckgasmotor, wobei eine Bürste an dem Antriebssystem befestigt ist.

In den Figuren und in der folgenden Beschreibung zu den anhand der Figuren erläuterten Ausführungsbeispielen der vorliegenden Erfindung werden teilweise für unterschiedliche Ausführungsbeispiele und für unterschiedliche Einzelteile die gleichen Bezugszeichen bei gleichen oder gleichartigen Teilen verwendet, um die Vergleichbarkeit der Ausführungsbeispiele und die Lesbarkeit zu vereinfachen.

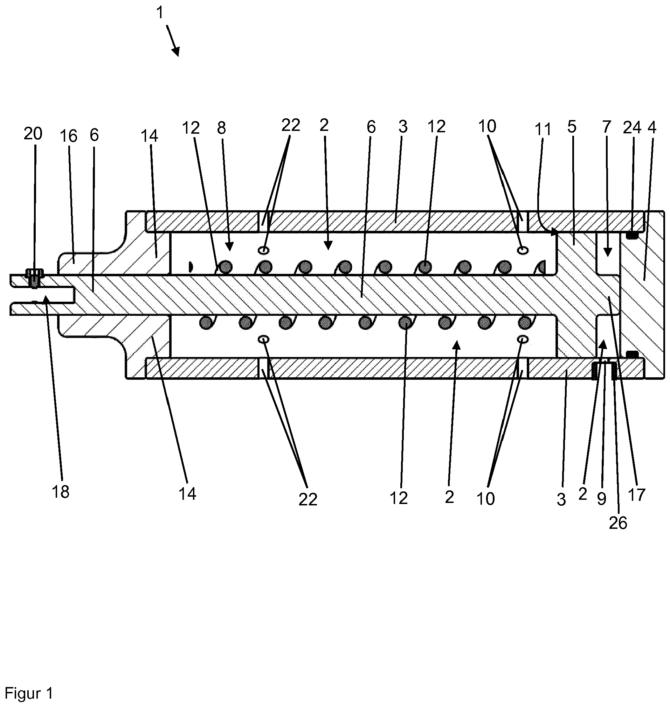

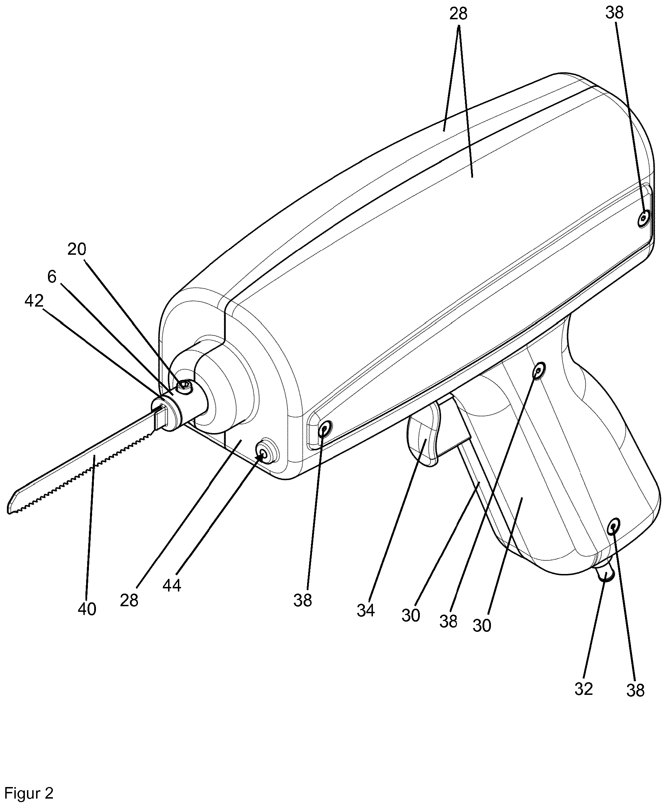

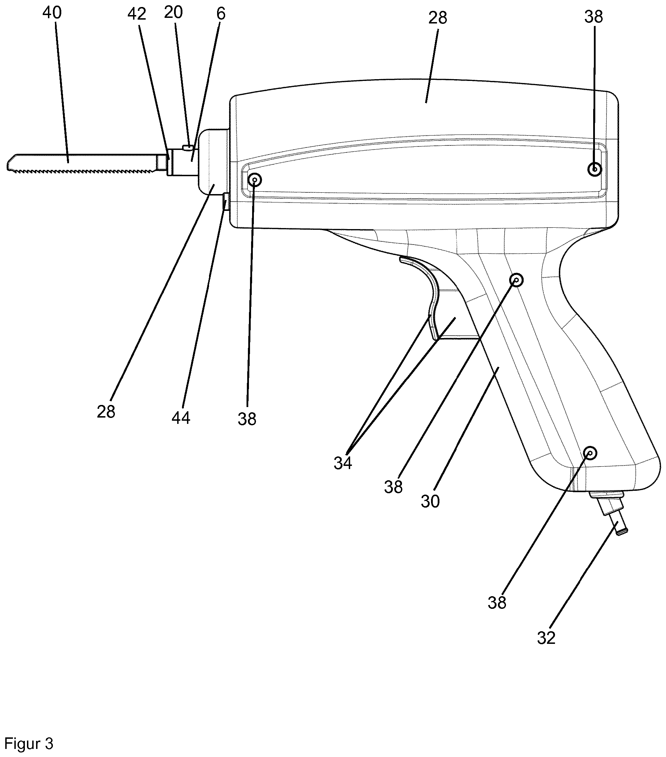

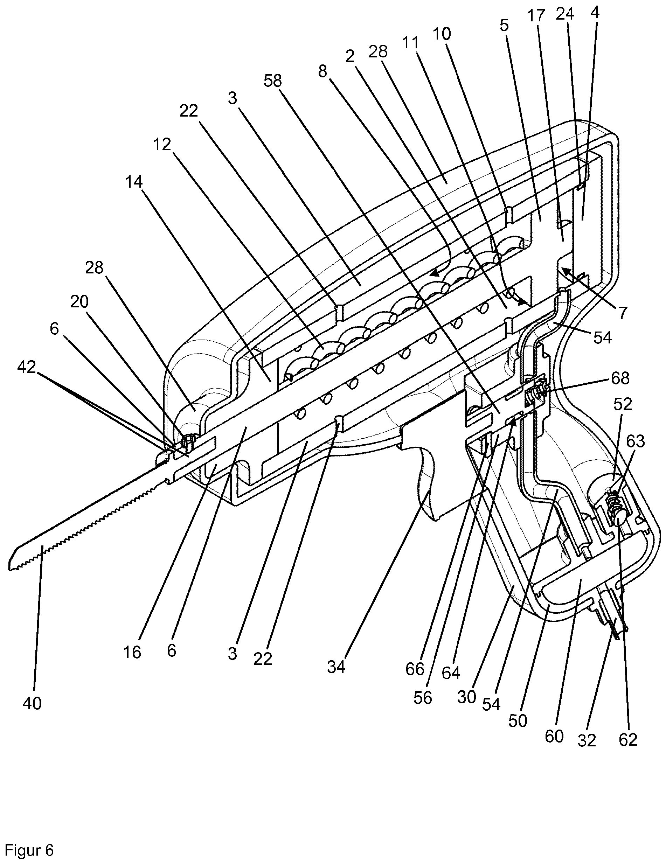

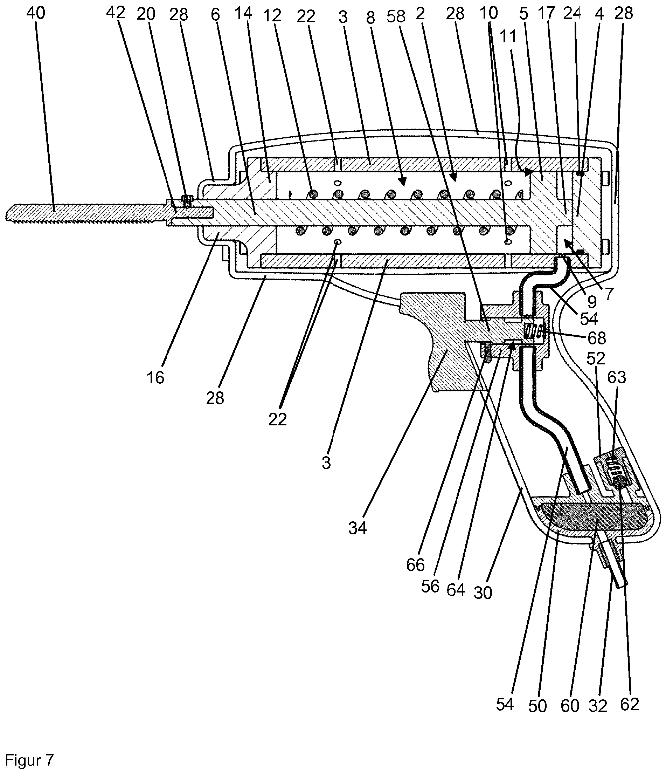

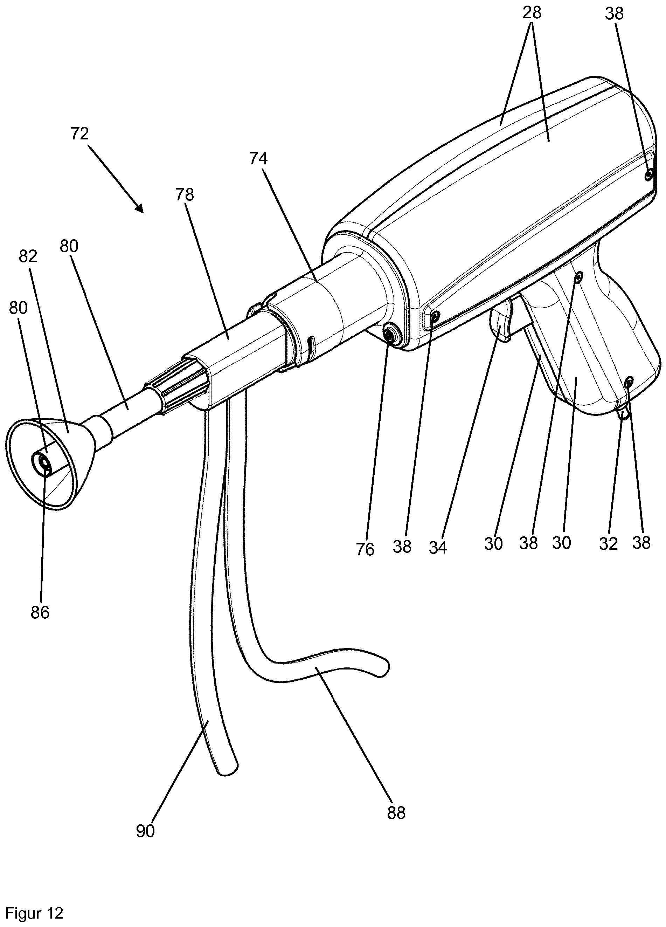

Figur 1 zeigt eine schematische Querschnittansicht eines beispielhaften erfindungsgemäßen Druckgasmotors 1 im Ruhezustand. Die Figuren 2 bis 5 zeigen verschiedene perspektivische Ansichten eines beispielhaften erfindungsgemäßen chirurgischen Antriebssystems mit einem erfindungsgemäßen Druckgasmotor 1. In den Figuren 6 bis 11 sind Querschnittansichten eines beispielhaften erfindungsgemäßen chirurgischen Antriebssystems mit einem erfindungsgemäßen Druckgasmotor 1 gezeigt, mit denen der Ablauf eines erfindungsgemäßen Verfahrens verdeutlicht wird. Die Figuren 12 bis 16 zeigen ein erfindungsgemäßes chirurgisches Antriebssystem mit einem erfindungsgemäßen Druckgasmotor und unterschiedlichen Aufsätzen, die in der Chirurgie anwendbar sind und die mit dem chirurgischen Antriebssystem anzutreiben sind. Der beispielhafte erfindungsgemäße Druckgasmotor nach Figur 1 könnte aber auch ohne weiteres auch zum Antreiben von anderen Werkzeugen oder auch anderen Aktoren auch im nichtmedizinischen Bereich eingesetzt werden.

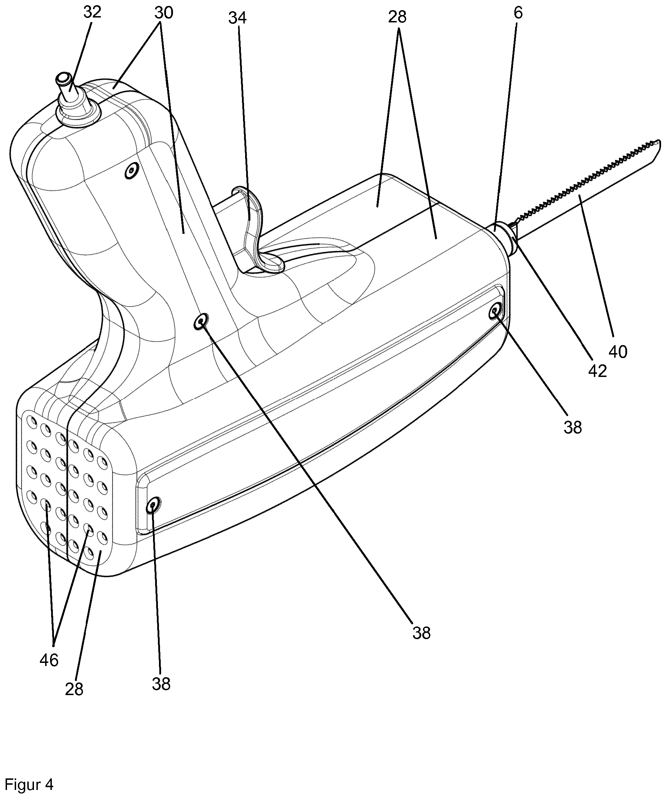

Die Vorderseiten der Druckgasmotoren und der chirurgischen Antriebssysteme sind in den Figuren 1 bis 3 und 5 bis 16 nach links und in Figur 4 nach rechts hinten ausgerichtet. Dementsprechend sind die Rückseiten der Druckgasmotoren und der chirurgischen Antriebssysteme sind in den Figuren 1 bis 3 und 5 bis 16 nach rechts und in Figur 4 nach links vorne ausgerichtet.

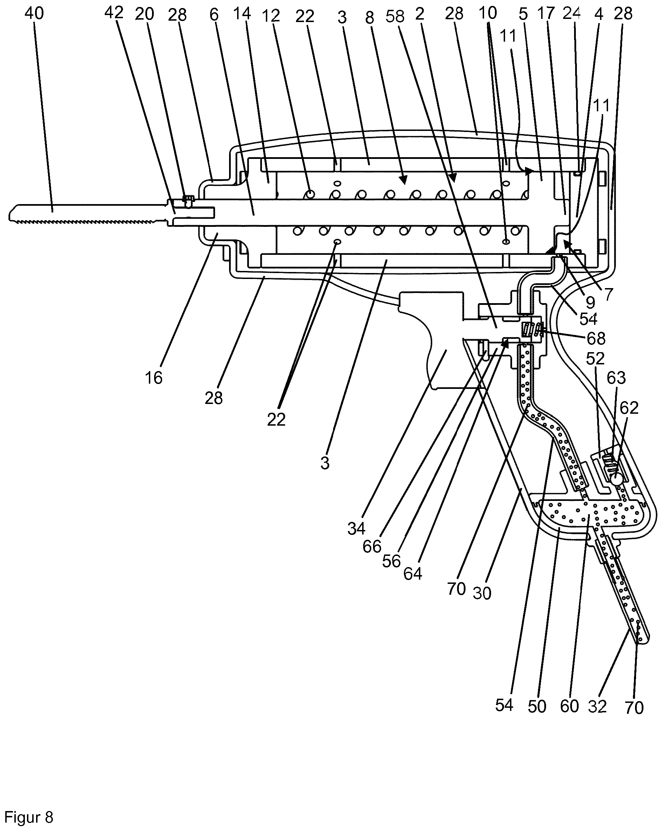

Der erste beispielhafte erfindungsgemäße Druckgasmotor 1 kann einen Hohlzylinder 2 aus Kunststoff aufweisen, der durch eine rohrförmige Mantelwand 3 aus Kunststoff begrenzt ist. An seinem rückseitigen Ende (in Figur 1 rechts) kann der Hohlzylinder 2 mit einem rückseitigen Verschluss 4 aus Kunststoff verschlossen sein. Der rückseitige Verschluss 4 definiert dann mit seiner vorderseitigen Oberfläche eine rückseitige Grundfläche des Hohlzylinders 2. Im Inneren des Hohlzylinders 2 ist ein Kolben 5 aus Kunststoff axial beweglich angeordnet. Axial bezieht sich dabei auf die Zylinderachse des Hohlzylinders 2. An der Vorderseite des Kolbens 5 (in Figur 1 links) kann eine Antriebsstange 6 aus Metall oder Kunststoff angeordnet sein, die fest mit dem Kolben 5 oder einteilig mit dem Kolben 5 ausgeführt sein kann. Der Kolben 5 kann den Hohlzylinder 2 in einen hinteren Innenraum 7 und einen vorderen Innenraum 8 unterteilen. Der hintere Innenraum 7 kann über eine Druckgaseinmündung 9 in der Mantelwand 3 mit einem Druckgas 70 (siehe Figuren 8 bis 11), wie zum Beispiel Druckluft oder CO₂-Gas aus einer CO₂-Patrone, beaufschlagt werden.

Der Kolben 5 kann einen zylindrischen Außenumfang aufweisen, der etwas kleiner ist als der Innendurchmesser des Hohlzylinders 2. Dadurch wird zwischen dem Kolben 5 und dem Hohlzylinder 2 ein kleiner Spalt 11 bereitgestellt. Durch diesen Spalt 11 kann ein Druckgas vom hinteren Innenraum 7 in den vorderen Innenraum 8 mit einem geringen Volumenstrom fließen. Dadurch kann der Kolben 5 auf einem Luftfilm beziehungsweise Druckgasfilm in dem Hohlzylinder 2 gleiten und tendiert weniger stark dazu zu blockieren. Der Spalt 11 muss dabei so klein gehalten werden, dass der Volumenstrom durch den Spalt 11 kleiner ist als der Volumenstrom durch die Druckgaseinmündung 9, damit sich im hinteren Innenraum 7 ein ausreichender Überdruck aufbauen lässt, mit dem der Kolben 5 anzutreiben ist. Bevorzugt ist hierzu der Leitungsquerschnitt des Spalts 11 zumindest um den Faktor zehn kleiner als der Leitungsquerschnitt der Druckgaseinmündung 9.

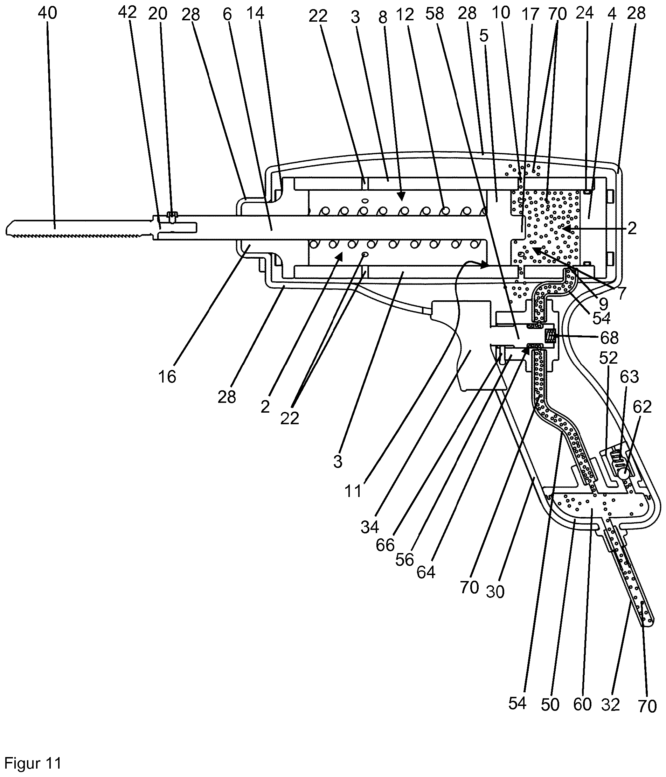

In einem hinteren Bereich der Mantelwand 3 können acht Belüftungsöffnungen 10 angeordnet sein, durch die das Druckgas 70 bei nach vorne ausgelenktem Kolben 5 aus dem hinteren Innenraum 7 entweichen kann (siehe Figur 11). Um die Antriebsstange 6 herum kann eine Druckfeder 12 im vorderen Innenraum 8 angeordnet sein. Die Druckfeder 12 kann gespannt werden, indem der Kolben 5 von dem Druckgas 70 in Richtung der Vorderseite gedrückt wird. Dabei kann sich die Druckfeder 12 an einem vorderseitigen Verschluss 14 aus Kunststoff abstützen. Der vorderseitige Verschluss 14 kann den Hohlzylinder 2 an seiner Vorderseite (in Figur 1 links) verschließen und definiert dadurch mit seiner rückseitigen Oberfläche eine vorderseitige Grundfläche des Hohlzylinders 2.

In dem vorderseitigen Verschluss 14 kann eine Durchführung angeordnet sein, in der die Antriebsstange 6 axial beweglich gelagert ist und durch die sich die Antriebsstange 6 hindurch aus dem Hohlzylinder 2 heraus erstreckt. Zur stabileren Führung der Antriebsstange 6 kann an dem vorderseitigen Verschluss 14 ein Führungsstutzen 16 angeordnet sein, der die Durchführung in dem vorderseitigen Verschluss 14 verlängert. In dem vorderseitigen Verschluss 14 kann zudem wenigstens eine gasdurchlässige Öffnung (nicht gezeigt) angeordnet sein, durch die der vordere Innenraum 8 gasdurchlässig mit der Umgebung verbunden ist.

Der hintere Innenraum 7 kann durch den Kolben 5, die Mantelwand 3 und den rückseitigen Verschluss 4 begrenzt sein. Der vordere Innenraum 8 kann durch den Kolben 5, die Mantelwand 3 und den vorderseitigen Verschluss 14 begrenzt sein.

An der Rückseite des Kolbens 5 kann ein Abstandhalter 17 angeordnet sein. Mit dem Abstandhalter 17 kann sichergestellt werden, dass der Kolben 5 die Druckgaseinmündung 9 niemals verschließt und so der hintere Innenraum 7 immer zur Druckgaseinmündung 9 hin offen ist. Der Abstandhalter 17 kann als Verlängerung der Antriebsstange 6 durch den Kolben 5 oder einteilig mit beiden ausgeführt sein.

An der Vorderseite der Antriebsstange 6, die aus dem Hohlzylinder 2 herausragt, kann ein Befestigungselement 18 in Form einer rechteckigen Vertiefung und einer Schraube 20 angeordnet sein. Alternativ kann das Befestigungselement 18 auch ein Innengewinde aufweisen. Mit dem Befestigungselement 18 kann ein Werkzeug mit der Antriebsstange 6 verbunden und angetrieben werden.

Im vorderen Bereich der Hohlzylinders 2, der niemals von dem Kolben 5 überfahren wird, können acht gasdurchlässige Öffnungen 22 in der Mantelwand 3 angeordnet sein. Durch diese gasdurchlässigen Öffnungen 22 bleibt der vordere Innenraum 8 mit der Umgebung des Druckgasmotors 1 verbunden. Hierdurch kann sich kein störender Gasdruck im vorderen Innenraum 8 aufbauen, der einer Bewegung des Kolbens 5 in Richtung des vorderseitigen Verschlusses 14 entgegenwirken würde. Alternativ oder zusätzlich können auch in dem vorderseitigen Verschluss 14 gasdurchlässige Öffnungen (nicht gezeigt) angeordnet sein. Es ist auch möglich keine gasdurchlässigen Öffnungen 22 vorzusehen. Der sich in dem vorderen Innenraum 8 ausbildende Gasdruck kann dann als Druckfederelement oder als zusätzliches Druckfederelement zur Rückstellung des Kolbens 5 in Richtung des rückseitigen Verschlusses 4 verwendet werden.

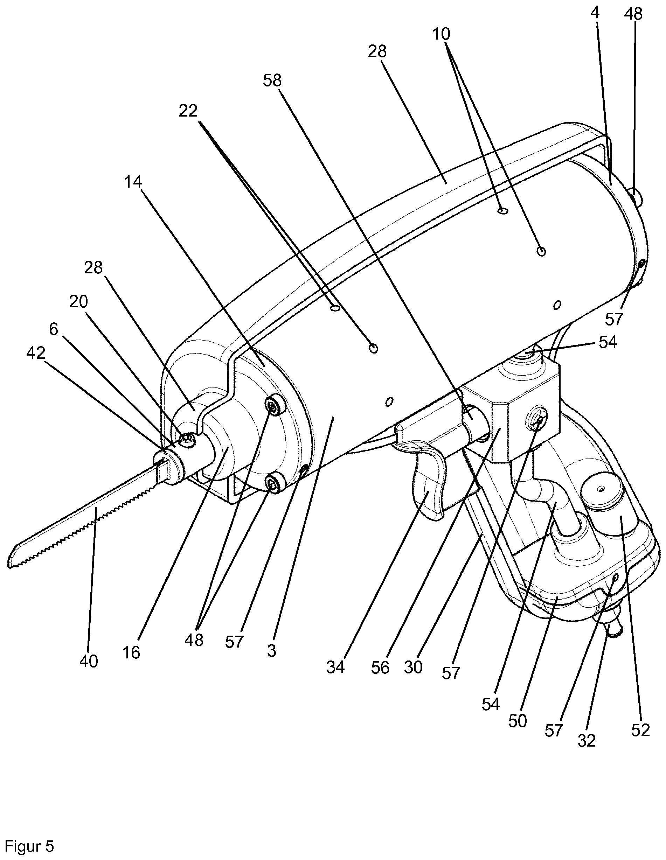

Um den hinteren Innenraum 7 abzudichten, kann der rückseitige Verschluss 4 mit einer umlaufenden Dichtung 24 aus Gummi gegen den Sitz in der Mantelwand 3 abgedichtet sein. Die umlaufende Dichtung 24 ist vorzugsweise ein O-Ring. An der Druckgaseinmündung 9 kann ein Anschluss 26 zum druckdichten Anschließen und zur Befestigung einer Druckgasleitung 54 (siehe Figuren 5 bis 11 und 13) angeordnet sein.

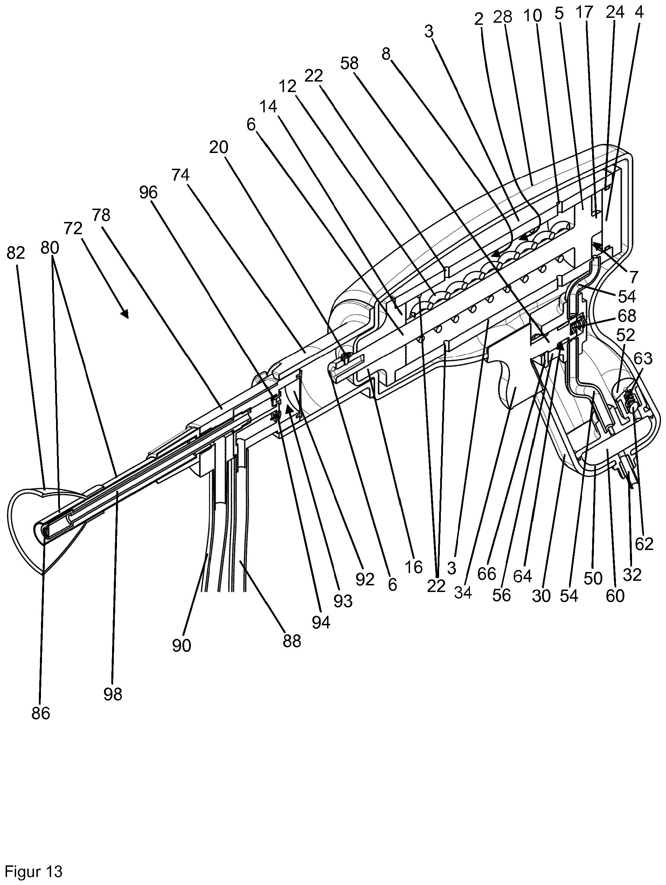

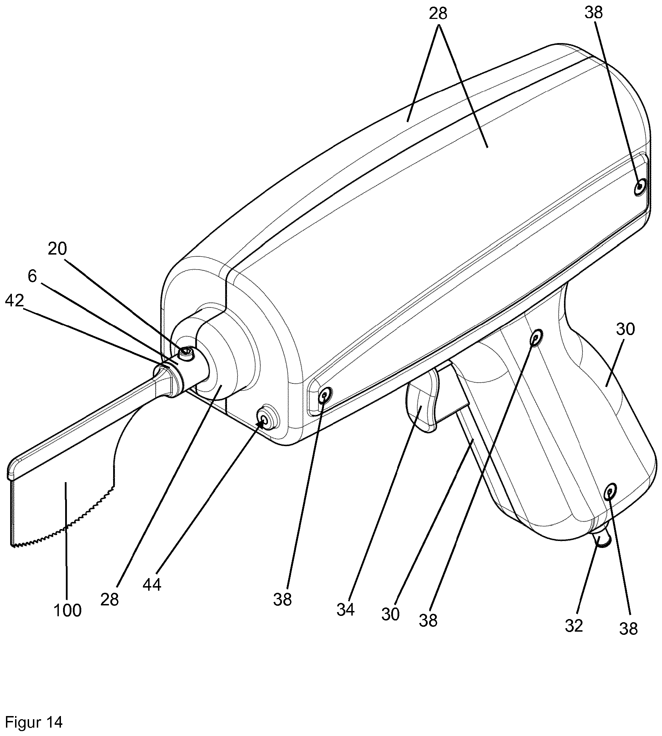

Die Figuren 2 bis 16 zeigen grafische Darstellungen eines chirurgischen Antriebssystems enthaltend den Druckgasmotor 1 nach Figur 1, wobei in den Figuren 2 bis 11 eine Säge 40 und in den Figuren 12 und 13 ein Lavage-Aufsatz 72 und in den Figuren 14 bis 16 unterschiedliche Werkzeuge an dem Antriebssystem befestigt sind.

Das chirurgische Antriebssystem kann ein zweiteiliges Gehäuse 28 aus Plastik oder Kunststoff aufweisen, das den Druckgasmotor 1 einhaust. Das Gehäuse 28 kann hierzu zwei im wesentlichen gleichförmige Halbschalen aufweisen. Ein Teil des Gehäuses 28 kann als Griff 30 ausgebildet sein, um das chirurgische Antriebssystem mit einer Hand halten zu können. Aus dem Griff kann ein Druckgasschlauch 32 führen, durch den ein Druckgas in das chirurgische Antriebssystem hinein und darin zum Druckgasmotor 1 geleitet werden kann. Der Druckgasschlauch 32 kann beispielsweise mit einem Druckluftreservoir beziehungsweise einer Pumpe zum Erzeugen von Druckluft verbunden sein oder werden. Alternativ kann eine Kartusche (nicht gezeigt) mit einem darin enthaltenen Druckgas, wie beispielsweise eine CO₂-Patrone, angeschlossen sein oder werden. Eine solche Druckgas-Kartusche kann auch in dem Gehäuse 28 angeordnet sein, ohne dass der Druckgasschlauch 32 aus dem Gehäuse 28 herausführt.

Das chirurgische Antriebssystem kann über einen Abzug 34 bedient werden, der im Bereich des Griffs 30 angeordnet ist. Die Teile des Gehäuses 28 können mit Nieten oder Schrauben 38 miteinander verbunden sein. Die Säge 40 kann ein Gegenbefestigungselement 42 mit einem rechteckigen Fortsatz oder einem Außengewinde aufweisen, das zu dem Befestigungselement 18 passt, so dass die Säge 40 mit dem Gegenbefestigungselement 42 in dem Befestigungselement 18 lösbar zu befestigen ist. Zur Sicherung der Säge 40 kann die Schraube 20 angezogen werden. An der Vorderseite des Gehäuses 28 kann ein Gegenbefestigungsmittel 44 in Form einer Bohrung mit einem Innengewinde angeordnet sein, mit dem komplexere Werkzeuge wie der Lavage-Aufsatz 72 an dem chirurgischen Antriebssystem befestigt werden können. An der Rückseite des Gehäuses 28 können mehrere Entlüftungsöffnungen 46 angeordnet sein, durch die Gas, das aus dem Druckgasmotor 1 im Inneren des Gehäuses 28 entweicht, austreten kann. Die Rückseite des Gehäuses 28 ist dabei bevorzugt, um an der Vorderseite im Bereich des Werkzeugs keinen Luftstrom beziehungsweise Gasstrom zu erzeugen.

Der rückseitige Verschluss 4 und der vorderseitige Verschluss 14 können mit Schrauben 48 an der Mantelwand 3 befestigt werden, um die Kraft des Gasdrucks im hinteren Innenraum 7 aber auch im vorderen Innenraum 8 aufzunehmen. Am Boden des Griffs 30 kann ein Sterilfilter 60 in einem Sterilfiltergehäuse 50 angeordnet sein. Der Druckgasschlauch 32 kann in den Sterilfilter 60 münden. Mit dem Sterilfilter 60 können Keime aus dem eingeleiteten Druckgas 70 entfernt werden, bevor diese in den Druckgasmotor 1 gelangen und anschließend an die Umgebung abgegeben werden können. Hierdurch kann der Operationssaal beziehungsweise die Umgebung des chirurgischen Antriebssystems hygienisch gehalten werden.

An dem Sterilfiltergehäuse 50 kann ein Überdruckventil 52 angeordnet sein, mit dem ein Überdruck in dem Sterilfiltergehäuse 50 abgeblasen werden kann. Damit kann verhindert werden, dass das Sterilfiltergehäuse 50 platzt. Des Weiteren kann an dem Sterilfiltergehäuse 50 die Druckgasleitung 54 angeschlossen sein. Das Druckgas 70, das durch die Druckgasschlauch 32 in den Sterilfilter 60 gedrückt wird, wird dann aus dem Sterilfilter 60 in die Druckgasleitung 54 innerhalb der Gehäuses 28 gedrückt.

In der Druckgasleitung 54 kann ein manuell bedienbares Ventilelement mit einem Ventilgehäuse 56 angeordnet sein. Das manuell bedienbare Ventilelement ist mit dem Abzug 34 bedienbar. An dem Ventilgehäuse 56, dem vorderseitigen Verschluss 14, dem rückseitigen Verschluss 4 und dem Sterilfiltergehäuse 50 können Bohrungen 57 angeordnet sein, mit denen die Nieten oder Schrauben 38 verbunden werden können, um das Gehäuse 28 an dem inneren Aufbau zu befestigen. Wenn Schrauben 38 verwendet werden, haben die Bohrungen 57 ein zu den Schrauben 38 passendes Innengewinde. Wenn Nieten 38 verwendet werden, können einfache Sacklöcher als Bohrungen 57 verwendet werden, in denen die Nieten 38 als Blindnieten befestigt werden. Alternativ oder zusätzlich ist es auch möglich, die beiden Hälften beziehungsweise Teile Gehäuses 28 direkt miteinander zu vernieten, verschrauben, verschweißen und/oder verkleben.

Das manuell bedienbare Ventilelement kann ein Ventil 58 in Form eines zylindrischen Stifts mit einer umlaufenden Ventilnut 64 aufweisen. Das Ventil 58 kann in axialer Richtung des zylindrischen Stifts beweglich im Ventilgehäuse 56 gelagert sein, das als Ventilsitz dient. Das Ventil 58 kann mit einem Bolzen 66 im Ventilgehäuse 56 gesichert sein. Hierzu kann das Ventil 58 an seiner Vorderseite eine Vertiefung aufweisen, in die der Bolzen 66 eingreifen kann. Das Ventil 58 kann im Ventilgehäuse 56 mit einer Feder 68 gelagert sein, mit der das Ventil 58 in den geschlossenen Zustand überführt wird. Gleichzeitig kann dadurch der Abzug 34 zur Vorderseite gedrückt werden. Im geschlossenen Zustand verschließt das Ventil 58 die Druckgasleitung 54 (siehe Figuren 6 bis 8). Dabei kann das Ventil 58 mit seinem äußeren Umfang die Druckgasleitung 54 im Ventilelement vollständig verschließen. Zum Öffnen des Ventilelements kann das Ventil 58 mit dem Abzug 34 gegen die Feder 68 in den Ventilsitz des Ventilgehäuses 56 gedrückt werden. Dabei kann sich die umlaufende Ventilnut 64 in die Druckgasleitung 54 bewegen und dadurch eine gasdurchlässige Verbindung bereitstellen.

Das Überdruckventil 52 kann mit einer Kugel 62 und einer Feder 63 aufgebaut werden, die die Kugel 62 in einen Kugelsitz des Überdruckventils 52 drückt. Alternativ kann auch eine Berstscheibe (nicht gezeigt) als Überdruckventil 52 verwendet werden.

In dem hinteren Innenraum 7 kann eine Zugfeder angeordnet sein (nicht gezeigt), die mit der Rückseite des Kolbens 5 und dem rückseitigen Verschluss 4 verbunden sein kann. die Zugfeder kann sich bei einer Bewegung des Kolbens 5 in Richtung des vorderseitigen Verschlusses 14 dehnen und dabei gespannt werden, so wie die Druckfeder 12 zusammengedrückt wird. Die in der Zugfeder gespeicherte Energie kann demzufolge analog zum Rückstellen des Kolbens 5 in Richtung des rückseitigen Verschlusses 4 verwendet werden. Die Zugfeder kann zusätzlich oder alternativ zur Druckfeder 12 im vorderen Innenraum 8 in dem hinteren Innenraum 7 angeordnet werden.

Der Betrieb des Druckgasmotors 1 beziehungsweise des chirurgischen Antriebssystems und damit ein beispielhaftes erfindungsgemäßes Verfahren wird im Folgenden Anhand der Figuren 8 bis 11 erläutert.

Ein Druckgas 70 wird über den Druckgasschlauch 32 durch den Sterilfilter 60 bis zum Ventilelement geleitet. Das Ventil 58 blockiert zunächst die Weiterleitung des Druckgases 70 durch die Druckgasleitung 54. Der Kolben 5 befindet sich in einer Ausgangsstellung, bei der die Druckfeder 12 den Kolben 5 in Richtung des rückseitigen Verschlusses 4 drückt beziehungsweise gedrückt hat. Der Kolben 5 ist zwischen den Belüftungsöffnungen 10 und dem rückseitigen Verschluss 4 angeordnet, so dass der hintere Innenraum 7 nicht in die Belüftungsöffnungen 10 mündet. Gleichzeitig sorgt der Abstandhalter 17 aber dafür, dass die Druckgaseinmündung 9 in den hinteren Innenraum 7 freiliegt. Diese Situation ist in Figur 8 gezeigt. Der Druck des Druckgases 70 reicht unter normalen Umständen jedoch nicht aus, um das Überdruckventil 52 zu öffnen, also die Kugel 62 gegen die Feder 63 aus dem Ventilsitz zu drücken oder um die Berstscheibe zu brechen.

Zum Betreiben des chirurgischen Antriebssystems wird das Ventil 58 mit dem Abzug 34 in das Ventilgehäuse 56 gedrückt und dadurch mit der Ventilnut 64 eine gasdurchlässige Verbindung in der Druckgasleitung 54 bereitgestellt. Während des Betriebs des Druckgasmotors 1 bleibt der Abzug 34 gedrückt und damit das Ventilelement geöffnet.

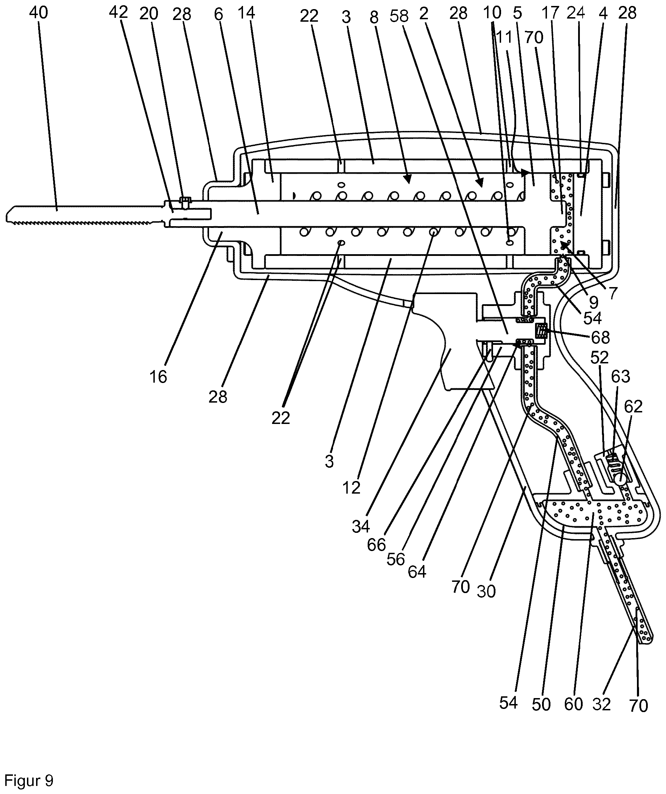

Druckgas 70 strömt durch die Druckgasleitung 54 und durch die Druckgaseinmündung 9 in den hinteren Innenraum 7 und beginnt, den Kolben 5 im Hohlzylinder 2 in Richtung des vorderseitigen Verschlusses 14 zu drücken. Dabei wird auch ein geringer Anteil des in den hinteren Innenraum 8 gedrückten Druckgases 70 durch den Spalt 11 zwischen dem Kolben 5 und der Mantelwand 3 gepresst, so dass sich zwischen dem Kolben 5 und der Mantelwand 3 ein dünner Gasfilm bildet, auf dem der Kolben 5 im Hohlzylinder 2 gleiten kann. Diese Situation ist in Figur 9 gezeigt. Der Spalt 11 ist nur sehr klein und daher in Figur 9 kaum erkennbar.

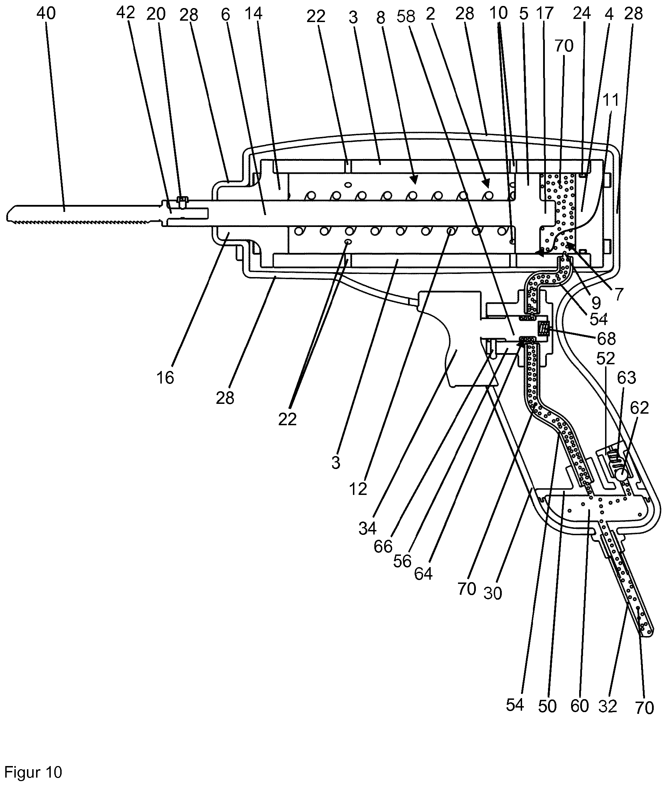

Das Druckgas 70 wird weiter in den hinteren Innenraum 7 gedrückt, so dass sich der Kolben 5 in Richtung des vorderseitigen Verschlusses 14 verschiebt und dadurch der hintere Innenraum 7 vergrößert wird. Dabei wird die Druckfeder 12, die sich am Kolben 5 und gegen den vorderseitigen Verschluss 14 abstützt, zusammengedrückt und gespannt. Mit dem Kolben 5 wird die Antriebsstange 6 aus dem Hohlzylinder 2 gedrückt und dabei die Säge 40 nach vorne bewegt. Diese Situation ist in Figur 10 gezeigt.

Der Kolben 5 wird von dem Druckgas 70 immer weiter in Richtung des vorderseitigen Verschlusses 14 gedrückt und passiert dabei die Belüftungsöffnungen 10. Der hintere Innenraum 7 ist dadurch mit den Belüftungsöffnungen 10 verbunden. Die Belüftungsöffnungen 10 haben zusammen einen größeren Leitungsquerschnitt als die Druckgaseinmündung 9. Dadurch entweicht das Druckgas 70 aus dem hinteren Innenraum 7 in Richtung der Umgebung und später durch die Entlüftungsöffnungen 46 aus dem Gehäuse 28. Aufgrund der Massenträgheit des Kolbens 5, der Antriebsstange 6, des Abstandhalters 17 und der Säge 40 überschwingt der Kolben 5 und die Belüftungsöffnungen 10 werden vollständig freigelegt. Der Druck im hinteren Innenraum 7 baut sich ab, da mehr Druckluft 70 aus dem hinteren Innenraum 7 entweicht, als durch die Druckgaseinmündung 9 nachströmen kann. Diese Situation ist in Figur 11 gezeigt.

Bei nachlassendem Druck im hinteren Innenraum 7 beschleunigt die gespannte Druckfeder 12 den Kolben 5 in Richtung des rückseitigen Verschlusses 4. Bei der Rückwärtsbewegung wird auch die Antriebsstange 6 mit der Säge 40 zurückbewegt. Der Kolben 5 passiert die Belüftungsöffnungen 10 und wird so lange in Richtung des rückseitigen Verschlusses gedrückt, bis sich durch einströmendes Druckgas 70 im hinteren Innenraum 7 wieder ein so großer Gasdruck aufbaut, dass die Bewegung erneut umgekehrt wird. Diese Situation ist in Figur 9 gezeigt.

Der Druckgasmotor 1 und das chirurgische Antriebssystem laufen so lange, wie das Ventilelement geöffnet bleibt. Der Kolben 5 und die Antriebsstange 6 oszillieren. Dabei wird auch die Säge 40 linear oszillierend bewegt und kann beispielsweise zum Sägen von Knochen verwendet werden.

Neben der Säge 40 kann das chirurgische Antriebssystem aber auch zu anderen Zwecken verwendet werden, wobei das dabei ablaufende Verfahren praktisch unverändert bleibt.

Die Figuren 12 und 13 zeigen das chirurgische Antriebssystem, das anstelle der Säge 40 mit dem Lavage-Aufsatz 72 verbunden ist. Der Lavage-Aufsatz 72 kann eine Halterung 74 aufweisen, die an dem chirurgischen Antriebssystem mit einem Befestigungsmittel 76 in Form einer Schraube befestigt sein kann. Hierzu kann die Schraube in das Gegenbefestigungsmittel 44 in Form einer Gewindebohrung geschraubt sein oder werden. Die Halterung 74 kann Teil eines Gehäuses 78 des Lavage-Aufsatzes 72 sein. Ein äußeres Rohr 80 kann sich aus dem Gehäuse 78 erstrecken und in einen Trichter 82 münden.

Im Inneren des Trichters 82 kann sich eine Düse 86 befinden, durch die eine medizinische Spülflüssigkeit mit Sprühstößen ausgestoßen werden kann. Eine Flüssigkeitszuleitung 88 und eine Absaugleitung 90 können in den Lavage-Aufsatz 72 führen. Durch die Flüssigkeitszuleitung 88 kann die medizinische Spülflüssigkeit in den Lavage-Aufsatz 72 gesaugt werden. Über die Absaugleitung 90 und den Trichter 82 können mit einem Unterdruck die gebrauchte Spülflüssigkeit sowie debridierte Körperflüssigkeiten und Gewebereste abgesaugt werden. Hierzu kann der Trichter 82 über das äußere Rohr 80 mit der Absaugleitung 90 verbunden sein.

Im Inneren des Lavage-Aufsatzes 70 kann sich eine Membran-Pumpe befinden, die mit der Antriebsstange 6 des chirurgischen Antriebssystems beziehungsweise des Druckgasmotors 1 anzutreiben ist. Hierzu kann die Membran-Pumpe eine Membran 92 aufweisen, die derart in dem Gehäuse 78 des Lavage-Aufsatzes 72 befestigt ist, dass beim Betrieb des Druckgasmotors 1 die Antriebsstange 6 wiederholt auf die Membran 92 schlägt. Spülflüssigkeit in einem Pumpraum 93, der von der Membran 92 auf seiner Rückseite begrenzt ist, wird durch den Schlag auf die Membran 92 durch ein Einwegventil 96 und ein inneres Rohr 98 durch die Düse 86 gepresst und dort ausgesprüht. Nach der elastischen Verformung der Membran 92 wird sich diese wieder zusammenziehen und dabei das Volumen des Pumpraums 93vergrößern. Dabei schließt sich das Einwegventil 96 und ein zweites Einwegventil 94, das an die Flüssigkeitszuleitung 88 angeschlossen ist, kann sich öffnen und Spülflüssigkeit in den Pumpraum 93 nachströmen. Die Einwegventile 94, 96 sind also gegensinnig mit dem Pumpraum 93 verbunden, so dass das Einwegventil 94 nur ein Einströmen in den Pumpraum 93 erlaubt, während das Einwegventil 96 nur ein Ausströmen aus dem Pumpraum 93 ermöglicht. Auf diese Weise kann das chirurgische Antriebssystem zum Antreiben des Lavage-Aufsatzes 72 zum Erzeugen von Sprühstößen verwendet werden.

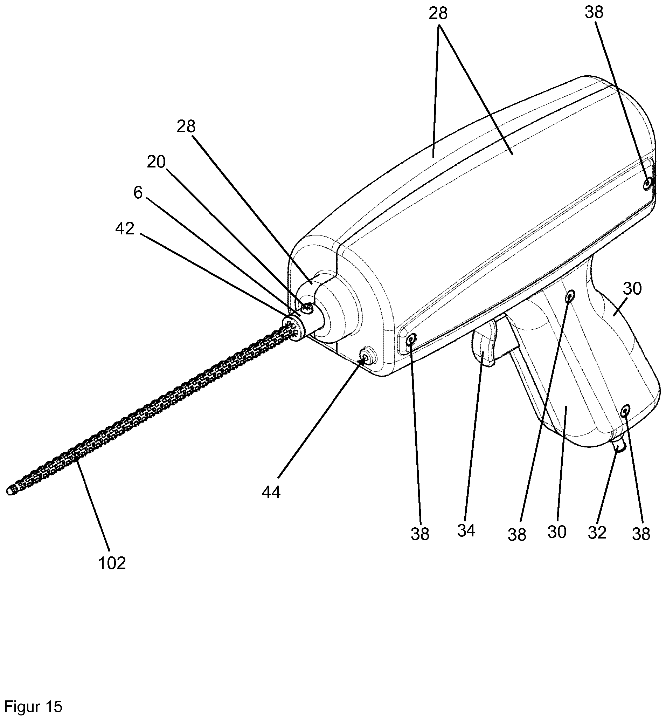

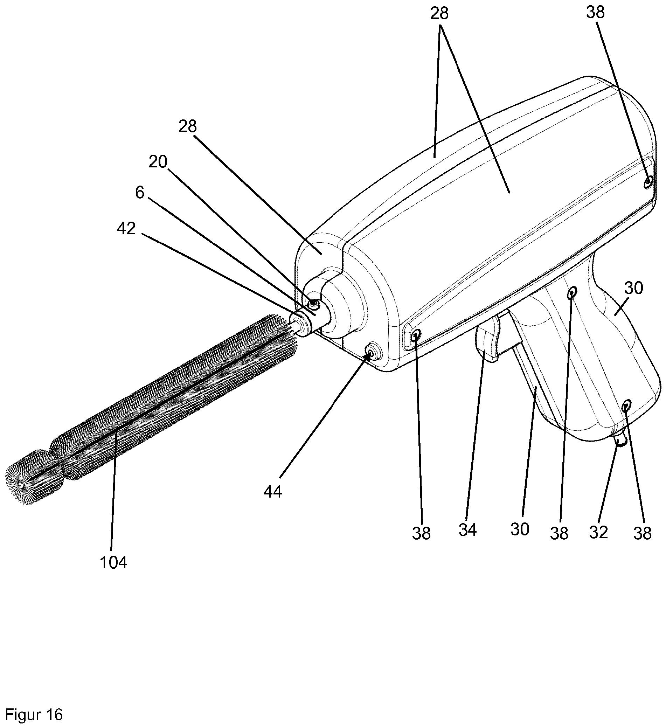

Alternativ zu der Säge 40 können also auch andere Werkzeuge wie andere Sägen 100 (Figur 14), Raspeln 102 (Figur 15) oder Bürsten 104 (Figur 16) mit dem Befestigungselement 20 an der Antriebsstange 6 verbunden werden oder es kann auch eine Flüssigkeitspumpe (siehe Figuren 12 und 13) angeschlossen und mit dem Druckgasmotor 1 betrieben werden, so dass mit dem Druckgasmotor 1 bei jedem Bewegungszyklus der Antriebstange 6 ein Sprühstoß mit einer medizinischen Spülflüssigkeit erzeugt wird. Auf diese Weise kann ein erfindungsgemäßes Lavage-System realisiert werden, das in den Figuren 12 und 13 dargestellt ist.

### Bezugszeichenliste

- 1: Druckgasmotor
- 2: Hohlzylinder
- 3: Mantelwand
- 4: Rückseitiger Verschluss
- 5: Kolben
- 6: Antriebsstange
- 7: Hinterer Innenraum
- 8: Vorderer Innenraum
- 9: Druckgaseinmündung
- 10: Belüftungsöffnung
- 11: Spalt
- 12: Druckfeder
- 14: Vorderseitiger Verschluss
- 16: Führungsstutzen
- 17: Abstandhalter
- 18: Befestigungselement
- 20: Schraube
- 22: Gasdurchlässige Öffnung
- 24: Dichtung
- 26: Anschluss für Druckgasleitung
- 28: Gehäuse
- 30: Griff
- 32: Druckgasschlauch
- 34: Abzug
- 38: Schraube oder Niete
- 40: Säge
- 42: Gegenbefestigungselement
- 44: Gegenbefestigungsmittel
- 46: Entlüftungsöffnung
- 48: Schraube
- 50: Sterilfiltergehäuse
- 52: Überdruckventil
- 54: Druckgasleitung
- 56: Ventilgehäuse
- 57: Bohrung
- 58: Ventil
- 60: Sterilfilter
- 62: Kugel
- 63: Feder
- 64: Ventilnut
- 66: Bolzen
- 68: Feder
- 70: Druckgas
- 72: Lavage-Aufsatz
- 74: Halterung
- 76: Befestigungsmittel
- 78: Gehäuse
- 80: Äußeres Rohr
- 82: Trichter
- 86: Düse
- 88: Flüssigkeitszuleitung
- 90: Absaugleitung
- 92: Membran
- 93: Pumpraum
- 94, 96: Einwegventil
- 98: Inneres Rohr
- 100: Säge
- 102: Raspel
- 104: Bürste

## Patentansprüche

1. Druckgasmotor (1) aufweisend
einen Hohlzylinder (2), der durch eine Mantelwand (3) begrenzt ist,
einen rückseitigen Verschluss (4) an einer rückseitigen Grundfläche des Hohlzylinders (2),
einen im Hohlzylinder (2) axial beweglichen Kolben (5), der an seiner Vorderseite mit einer Antriebsstange (6) verbunden ist, die aus dem Hohlzylinder (2) herausragt, wobei der Kolben (5) den Hohlzylinder (2) in zwei Innenräume trennt, und zwar in einen hinteren Innenraum (7) des Hohlzylinders (2), der durch den Kolben (5), die Mantelwand (3) des Hohlzylinders (2) und den rückseitigen Verschluss (4) begrenzt ist, und
einen vorderen Innenraum (8) des Hohlzylinders (2), der durch den Kolben (5) und die Mantelwand (3) des Hohlzylinders (2) und eine vorderseitige Grundfläche des Hohlzylinders (2) begrenzt ist,
mindestens eine Druckgaseinmündung (9), die in den hinteren Innenraum (7) des Hohlzylinders (2) einmündet,
mindestens eine Belüftungsöffnung (10) in der Mantelwand (3) des Hohlzylinders (2), wodurch der hintere Innenraum (7) des Hohlzylinders (2) mit der umgebenden Atmosphäre während des Betriebs des Druckgasmotors (1) gasdurchlässig verbunden oder verbindbar ist, wobei die mindestens eine Belüftungsöffnung (10) vom axial beweglichen Kolben (5) während des Betriebs des Druckgasmotors (1) zum hinteren Innenraum (7) hin durch die Bewegung des Kolbens (5) periodisch freilegbar ist,
mindestens eine im vorderen Innenraum (8) angeordnete Druckfeder (12), wobei sich die mindestens eine Druckfeder (12) während des Betriebs des Druckgasmotors (1) zumindest zeitweise an der vorderseitigen Grundfläche des Hohlzylinders (2) abstützt, und/oder
mindestens eine im hinteren Innenraum (7) angeordnete Zugfeder, wobei die mindestens eine Zugfeder mit dem Kolben (5) und mit dem rückseitigen Verschluss (4) des Hohlzylinders (2) verbunden ist, wobei
die mindestens eine Druckfeder (12) den Kolben (5) so weit in Richtung des rückseitigen Verschlusses (4) drückt und/oder die mindestens eine Zugfeder den Kolben (5) so weit in Richtung des rückseitigen Verschlusses (4) zieht, dass der hintere Innenraum (7) des Hohlzylinders (2) gegenüber der mindestens einen Belüftungsöffnung (10) vom Kolben (5) verschlossen ist und der hintere Innenraum (7) mit der Druckgaseinmündung (9) verbunden ist, wenn im vorderen Innenraum (8) des Hohlzylinders (2) und im hinteren Innenraum (7) des Hohlzylinders (2) der gleiche Druck herrscht, und wobei zwischen dem Kolben (5) und der Mantelwand (3) des Hohlzylinders (2) ein gasdurchlässiger Spalt (11) vorhanden ist, durch den der hintere Innenraum (7) und der vordere Innenraum (8) gasdurchlässig miteinander verbunden sind.

2. Druckgasmotor (1) nach Anspruch 1, **dadurch gekennzeichnet, dass** der Druckgasmotor (1) einen vorderseitigen Verschluss (14) der vorderseitigen Grundfläche des Hohlzylinders (2) aufweist, wobei der vorderseitige Verschluss (14) eine Durchbrechung aufweist, insbesondere eine zentrale axiale Durchbrechung aufweist, durch die die Antriebsstange (6) hindurch geführt ist, und wobei der vordere Innenraum (8) des Hohlzylinders (2) durch den Kolben (5), die Innenwand des Hohlzylinders (2) und den vorderseitigen Verschluss (14) begrenzt ist, wobei bevorzugt der vorderseitige Verschluss (14) zumindest eine gasdurchlässige Öffnung aufweist.

3. Druckgasmotor (1) nach Anspruch 1 oder 2, **dadurch gekennzeichnet, dass** der Kolben (5) den Hohlzylinder (2) in zwei voneinander separate Innenräume trennt, bevorzugt der Kolben (5) den Hohlzylinder (2) nicht gasdicht in zwei voneinander separate Innenräume trennt.

4. Druckgasmotor (1) nach einem der vorangehenden Ansprüche, **dadurch gekennzeichnet, dass**
bei Einspeisen eines unter Druck stehenden Druckgases (70) durch die mindestens eine Druckgaseinmündung (9) in den hinteren Innenraum (7) des Hohlzylinders (2) der Kolben (5) durch periodische Wechsel der Einwirkung des Druckgases (70) und der umgebenden Atmosphäre im hinteren Innenraum (7) eine oszillierende Bewegung der Antriebsstange (6) bewirkt, wobei bevorzugt
die mindestens eine Druckfeder (12) und/oder die mindestens eine Zugfeder durch die Bewegung des Kolbens (5) in Richtung der vorderen Grundfläche des Hohlzylinders (2) angetrieben von dem Druckgas (70) zu spannen ist und der Kolben (5) von der gespannten mindestens einen Druckfeder (12) und/oder der gespannten mindestens einen Zugfeder in Richtung der rückseitigen Grundfläche zu drücken ist, wenn der hintere Innenraum (7) zur umgebenden Atmosphäre geöffnet ist.

5. Druckgasmotor (1) nach einem der vorangehenden Ansprüche, **dadurch gekennzeichnet, dass**
der Kolben (5) einen Durchmesser von größer oder gleich 20 mm hat, bevorzugt von größer oder gleich 30 mm und besonders bevorzugt von größer oder gleich 35 mm hat.

6. Druckgasmotor (1) nach einem der vorangehenden Ansprüche, **dadurch gekennzeichnet, dass**
an der Vorderseite der zumindest einen Antriebsstange (6) ein Befestigungselement (18), insbesondere ein Gewinde, angeordnet ist, über das ein Werkzeug, wie eine Säge (40, 100), eine Raspel (102) oder eine Bürste (104), mit einem zum Befestigungselement (18) passenden Gegenbefestigungselement (44), insbesondere einem zum Gewinde passenden Gegengewinde, an der zumindest einen Antriebsstange (6) befestigbar ist.

7. Druckgasmotor (1) nach einem der vorangehenden Ansprüche, **dadurch gekennzeichnet, dass**
die den Hohlzylinder (2) begrenzende Mantelwand (3), der Kolben (5), der rückseitige Verschluss (4) und, sofern vorhanden, auch ein vorderseitiger Verschluss (14), aus einem Kunststoff gefertigt sind, insbesondere aus einem thermoplastischen Kunststoff gefertigt sind, vorzugsweise durch Spritzgießen geformt sind.

8. Druckgasmotor (1) nach einem der vorangehenden Ansprüche, **dadurch gekennzeichnet, dass**
die mindestens eine Druckfeder (12) eine Spiralfeder ist, wobei die Spiralfeder die Antriebsstange (6) umschließt, wobei vorzugsweise keine Zugfeder in dem hinteren Innenraum (7) angeordnet ist.

9. Druckgasmotor (1) nach einem der vorangehenden Ansprüche, **dadurch gekennzeichnet, dass**
der Spalt (11) eine Querschnittsfläche aufweist, die weniger als 1 % der Querschnittsfläche des Hohlzylinders (2) senkrecht zur Zylinderachse beträgt, besonders bevorzugt weniger als 0,5 %, ganz besonders bevorzugt weniger als 0,1 % der Querschnittsfläche des Hohlzylinders (2) senkrecht zur Zylinderachse beträgt.

10. Druckgasmotor (1) nach einem der vorangehenden Ansprüche, **dadurch gekennzeichnet, dass**
die Lage der mindestens einen Belüftungsöffnung (10) in der Mantelwand (3) entlang der axialen Erstreckung des Hohlzylinders (2) den Hub des Kolbens (5) mit der Antriebsstange (6) bestimmt.

11. Chirurgisches Antriebssystem aufweisend einen Druckgasmotor (1) nach einem der vorangehenden Ansprüche sowie ein Ventilelement, insbesondere ein manuell bedienbares Ventilelement, wobei das Ventilelement in einer Druckgasleitung (54) angeordnet ist, die mit einer der mindestens einen Druckgaseinmündung (9) verbunden ist und die mit einem Druckgasreservoir verbindbar oder verbunden ist, so dass mit dem Ventilelement die Verbindung zu dem Druckgasreservoir unterbrechbar und/oder der Druck an der mindestens einen Druckgaseinmündung (9) einstellbar ist.

12. Chirurgisches Antriebssystem nach Anspruch 11, **dadurch gekennzeichnet, dass** das chirurgische Antriebssystem einen Griff (30) aufweist, mit dem es in einer Hand haltbar ist und das Ventilelement mit einem Abzug (34) am Griff (30) mit der gleichen Hand bedienbar ist.

13. Chirurgisches Antriebssystem nach Anspruch 11 oder 12, **dadurch gekennzeichnet, dass**
in der Druckgasleitung (54) ein Sterilfilter (60) angeordnet ist.

14. Medizinisches Lavage-System zum Debridement von Weichgewebe und/oder Knochengewebe aufweisend einen Druckgasmotor (1) nach einem der Ansprüche 1 bis 10 oder ein chirurgisches Antriebssystem nach einem der Ansprüche 11 bis 13 oder medizinische Vorrichtung zum Bürsten, Raspeln oder Sägen von Weichgewebe und/oder Knochengewebe aufweisend einen Druckgasmotor (1) nach einem der Ansprüche 1 bis 10 oder ein chirurgisches Antriebssystem nach einem der Ansprüche 11 bis 13, wobei vorzugsweise an der Antriebsstange (6) der medizinischen Vorrichtung ein Werkzeug, insbesondere eine Säge (40, 100), eine Raspel (102) oder eine Bürste (104) befestigt ist.

15. Verfahren zum Betreiben eines Druckgasmotors (1), bei dem ein Kolben (5) linear und axial in einem Hohlzylinder (2) oszilliert, wobei der Kolben (5) mit einer Antriebsstange (6) verbunden ist, die aus dem Hohlzylinder (2) herausragt und der Kolben (5) die Antriebsstange (6) antreibt, wobei das Verfahren die folgenden chronologischen Schritte umfasst:
A) der Kolben (5) begrenzt in einem Ausgangszustand einen hinteren Innenraum (7) des Hohlzylinders (2), der bis auf mindestens eine Druckgaseinmündung (9) gegenüber der Umgebung des Druckgasmotors (1) geschlossen ist;
B) Einleiten eines Druckgases (70) in den hinteren Innenraum (7) des Hohlzylinders (2) durch die mindestens eine Druckgaseinmündung (9);
C) Drücken des Kolbens (5) mit der Antriebsstange (6) in Richtung einer Vorderseite des Hohlzylinders (2) unter Vergrößerung des hinteren Innenraums (7) durch den Gasdruck des Druckgases (70) im hinteren Innenraum (7);
D) Spannen mindestens einer Druckfeder (12) und/oder mindestens einer Zugfeder durch die Bewegung des Kolbens (5);
E) Freilegen mindestens einer Belüftungsöffnung (10) in einer Mantelwand (3) des Hohlzylinders (2) zum hinteren Innenraum (7) durch die Bewegung des Kolbens (5), wobei die mindestens eine Belüftungsöffnung (10) direkt in den hinteren Innenraum (7) geöffnet wird;
F) Ausströmen des Druckgases (70) aus dem hinteren Innenraum (7) durch die mindestens eine Belüftungsöffnung (10),
G) Rückstellen des Kolbens (5) durch eine Krafteinwirkung der mindestens einen Druckfeder (12) und/oder mindestens einen Zugfeder auf den Kolben (5); und
H) Verschließen der mindestens einen Belüftungsöffnung (10) gegen den hinteren Innenraum (7) durch die Bewegung des Kolbens (5), wobei
der Kolben (5) in dem Hohlzylinder (2) auf einem Gasfilm des Druckgases (70) gleitet, der durch einen Spalt (11) zwischen dem Kolben (5) und der Innenwand des Hohlzylinders (2) strömt.

16. Verfahren nach Anspruch 15, **dadurch gekennzeichnet, dass** das Verfahren mit einem Druckgasmotor (1) nach einem der Ansprüche 1 bis 10 oder mit einem chirurgischen Antriebssystem nach einem der Ansprüche 11 bis 13 oder mit einem Lavage-System oder einer medizinischen Vorrichtung zum Bürsten, Raspeln oder Sägen von Weichgewebe und/oder Knochengewebe nach Anspruch 14 durchgeführt wird.

17. Verfahren nach einem der Ansprüche 15 oder 16, **dadurch gekennzeichnet, dass** mit der Bewegung der Antriebsstange (6) beziehungsweise des Kolbens (5) ein Werkzeug oder eine Pumpe angetrieben wird, insbesondere ein medizinisches Werkzeug, wie eine Säge (40, 100), eine Raspel (102) oder eine Bürste (104), oder ein Lavage-Aufsatz (72) angetrieben wird.

## Claims

1. A compressed-gas motor (1) comprising
a hollow cylinder (2) delimited by a cylinder jacket wall (3),
a rear closure (4) at a rear base of the hollow cylinder (2),
a plunger (5) which is axially movable in the hollow cylinder (2) and which is connected at a front end of the plunger (5) with a drive rod (6) which projects out of the hollow cylinder (2), wherein the plunger (5) divides the hollow cylinder (2) into two inner chambers, specifically into
a back inner chamber (7) of the hollow cylinder (2) being delimited by the plunger (5), the cylinder jacket wall (3) of the hollow cylinder (2) and the rear closure (4), and
a front inner chamber (8) of the hollow cylinder (2) being delimited by the plunger (5) and the cylinder jacket wall (3) of the hollow cylinder (2) and a front base of the hollow cylinder (2),
at least one compressed-gas port (9), which leads into the back inner chamber (7) of the hollow cylinder (2),
at least one ventilation opening (10) in the cylinder jacket wall (3) of the hollow cylinder (2), whereby the back inner chamber (7) of the hollow cylinder (2) is connected or connectable for passage of gas with the surrounding atmosphere during operation of the compressed-gas motor (1), wherein the at least one ventilation opening (10) is periodically openable towards the back inner chamber (7) by the axially movable plunger (5) during operation of the compressed-gas motor (1) by movement of the plunger (5),
at least one compression spring (12) arranged in the front inner chamber (8), wherein the at least one compression spring (12) rests during operation of the compressed-gas motor (1) at least temporarily against the front base of the hollow cylinder (2), and/or
at least one tension spring arranged in the back inner chamber (7), wherein the at least one tension spring is connected with the plunger (5) and with the rear closure (4) of the hollow cylinder (2), wherein
the at least one compression spring (12) urges the plunger (5) to such an extent towards the rear closure (4) and/or the at least one tension spring draws the plunger (5) to such an extent towards the rear closure (4) that the back inner chamber (7) of the hollow cylinder (2) is closed relative to the at least one ventilation opening (10) by the plunger (5) and the back inner chamber (7) is connected with the compressed-gas port (9) when the same pressure prevails in the front inner chamber (8) of the hollow cylinder (2) and in the back inner chamber (7) of the hollow cylinder (2), and wherein a gap (11) allowing passage of gas is present between the plunger (5) and the cylinder jacket wall (3) of the hollow cylinder (2), the back inner chamber (7) and the front inner chamber (8) being connected together by said gap for passage of gas.

2. The compressed-gas motor (1) according to Claim 1, **characterized in that**
the compressed-gas motor (1) includes the front closure (14) of the front base of the hollow cylinder (2), wherein the front closure (14) includes a hole, in particular a central axial hole, through which the drive rod (6) is passed and wherein the front inner chamber (8) of the hollow cylinder (2) is delimited by the plunger (5), the internal wall of the hollow cylinder (2) and the front closure (14), wherein the front closure (14) preferably includes at least one opening allowing passage of gas.

3. The compressed-gas motor (1) according to Claim 1 or 2, **characterized in that** the plunger (5) divides the hollow cylinder (2) into two inner chambers separate from one another, preferably the plunger (5) divides the hollow cylinder (2) into two inner chambers separate from one another in non-gas-tight manner.

4. The compressed-gas motor (1) according to any one of the preceding claims, **characterized in that**
on feed-in of a pressurized compressed gas (70) through the at least one compressed-gas port (9) into the back inner chamber (7) of the hollow cylinder (2), the plunger (5) causes oscillating movement of the drive rod (6) by periodic changing of the action of the compressed gas (70) and of the surrounding atmosphere in the back inner chamber (7), wherein preferably
the at least one compression spring (12) and/or the at least one tension spring is able to be tensioned by the movement of the plunger (5) towards the front base of the hollow cylinder (2), driven by the compressed gas (70), and the plunger (5) is able to be urged by the tensioned at least one compression spring (12) and/or the tensioned at least one tension spring towards the rear base when the back inner chamber (7) is open to the surrounding atmosphere.

5. The compressed-gas motor (1) according to any one of the preceding claims, **characterized in that**
the plunger (5) has a diameter of greater than or equal to 20 mm, preferably of greater than or equal to 30 mm and particularly preferably of greater than or equal to 35 mm.

6. The compressed-gas motor (1) according to any one of the preceding claims, **characterized in that**
a fastening element (18), in particular a thread, is arranged at the front end of the at least one drive rod (6), by which fastening element a tool, such as a saw (40, 100), a rasp (102) or a brush (104), is fastenable by a counter-fastening element (44) matching the fastening element (18), in particular a counter-thread matching the thread, on the at least one drive rod (6).

7. The compressed-gas motor (1) according to any one of the preceding claims, **characterized in that**
the cylinder jacket wall (3) delimiting the hollow cylinder (2), the plunger (5), the rear closure (4) and, where present, also a front closure (14), are made from a plastics material, in particular from a thermoplastic, preferably formed by injection molding.

8. The compressed-gas motor (1) according to any one of the preceding claims, **characterized in that**
the at least one compression spring (12) is a spiral spring, wherein the spiral spring encloses the drive rod (6), wherein preferably no tension spring is arranged in the back inner chamber (7).

9. The compressed-gas motor (1) according to any one of the preceding claims, **characterized in that**
the gap (11) allowing passage of gas has a cross-sectional area which amounts to less than 1 % of the cross-sectional area of the hollow cylinder (2) perpendicular to the cylinder axis, particularly preferably amounts to less than 0.5 %, very particularly preferably to less than 0.1 % of the cross-sectional area of the hollow cylinder (2) perpendicular to the cylinder axis.

10. The compressed-gas motor (1) according to Claim 11, **characterized in that**
the position of the at least one ventilation opening (10) in the cylinder jacket wall (3) along the axial extent of the hollow cylinder (2) determines the stroke of the plunger (5) with the drive rod (6).

11. A surgical drive system including a compressed-gas motor (1) according to any one of the preceding claims and a valve element, in particular a manually actuatable valve element, wherein the valve element is arranged in a compressed-gas line (54) which is connected with one of the at least one compressed-gas port (9) and which is connectable or connected with a compressed-gas reservoir, such that the connection to the compressed-gas reservoir is interruptible and/or the pressure at the at least one compressed-gas port (9) is adjustable with the valve element.

12. A surgical drive system according to Claim 11, **characterized in that**
the surgical drive system has a handle (30), by which it is holdable in a hand and the valve element is actuatable with the same hand by means of a trigger (34) on the handle (30).

13. The surgical drive system according to Claim 11 or 12, **characterized in that**
a sterile filter (60) is arranged in the compressed-gas line (54).

14. A medical lavage system for debridement of soft tissue and/or bone tissue, including a compressed-gas motor (1) according to any one of Claims 1 to 10 or a surgical drive system according to any one of Claims 11 to 13 or
a medical device for brushing, rasping or sawing soft tissue and/or bone tissue, including a compressed-gas motor (1) according to any one of Claims 1 to 10 or a surgical drive system according to any one of Claims 11 to 13, wherein preferably, a tool is fastened to the drive rod (6) of the medical device, in particular a saw (40, 100), a rasp (102) or a brush (104) is fastened to the drive rod (6) of the medical device.

15. A method for operating a compressed-gas motor (1), in which a plunger (5) oscillates in linear and axial manner in a hollow cylinder (2), wherein the plunger (5) is connected with a drive rod (6), which projects out of the hollow cylinder (2) and the plunger (5) drives the drive rod (6), wherein the method comprises the following chronological steps:
A) in an initial state, the plunger (5) delimits a back inner chamber (7) of the hollow cylinder (2), which is closed relative to the surroundings of the compressed-gas motor (1) apart from at least one compressed-gas port (9);
B) Introducing a compressed gas (70) into the back inner chamber (7) of the hollow cylinder (2) through the at least one compressed-gas port (9);
C) Pushing the plunger (5) together with the drive rod (6) towards a front end of the hollow cylinder (2) with enlargement of the back inner chamber (7) by means of the gas pressure of the compressed gas (70) in the back inner chamber (7);
D) Tensioning at least one compression spring (12) and/or at least one tension spring by movement of the plunger (5);
E) Opening up at least one ventilation opening (10) in a cylinder jacket wall (3) of the hollow cylinder (2) to the back inner chamber (7) by the movement of the plunger (5), wherein the at least one ventilation opening (10) is opened directly into the back inner chamber (7);
F) Flowing of the compressed gas (70) out of the back inner chamber (7) through the at least one ventilation opening (10),
G) Returning the plunger (5) by application of the force of the at least one compression spring (12) and/or at least one tension spring to the plunger (5); and
H) Closing the at least one ventilation opening (10) relative to the back inner chamber (7) through the movement of the plunger (5), wherein
the plunger (5) slides in the hollow cylinder (2) on a gas film of the compressed gas (70), which flows through a gap (11) between the plunger (5) and the internal wall of the hollow cylinder (2).

16. The method according to Claim 15, **characterized in that**
the method is carried out with a compressed-gas motor (1) according to any one of Claims 1 to 10 or with a surgical drive system according to any one of Claims 11 to 13 or with a lavage system or a medical device for brushing, rasping or sawing soft tissue and/or bone tissue according to Claim 14.

17. The method according to either one of Claims 15 or 16, **characterized in that**
a tool or a pump is driven by the movement of the drive rod (6) or the plunger (5), in particular a medical tool, such as a saw (40, 100), a rasp (102) or a brush (104), or a lavage attachment (72).

## Revendications

1. Moteur à gaz comprimé (1) présentant
un cylindre creux (2) délimité par une paroi formant enveloppe (3),
une fermeture côté arrière (4) sur une surface formant base côté arrière du cylindre creux (2),
un piston (5) pouvant se déplacer axialement dans le cylindre creux (2), lequel piston est relié, sur son côté avant, à une tige d'entraînement (6) qui fait saillie hors du cylindre creux (2), dans lequel le piston (5) sépare le cylindre creux (2) en deux espaces intérieurs, à savoir en un espace intérieur arrière (7) du cylindre creux (2), lequel est délimité par le piston (5), la paroi formant enveloppe (3) du cylindre creux (2) et la fermeture côté arrière (4), et
en un espace intérieur avant (8) du cylindre creux (2), lequel est délimité par le piston (5) et la paroi formant enveloppe (3) du cylindre creux (2) et une surface formant base côté avant du cylindre creux (2),
au moins une embouchure de gaz comprimé (9) qui débouche dans l'espace intérieur arrière (7) du cylindre creux (2),
au moins une ouverture d'aération (10) dans la paroi formant enveloppe (3) du cylindre creux (2), moyennant quoi l'espace intérieur arrière (7) du cylindre creux (2) est ou peut être raccordé à l'atmosphère environnante de manière perméable aux gaz pendant le fonctionnement du moteur à gaz comprimé (1), dans lequel l'au moins une ouverture d'aération (10) peut être dégagée périodiquement du piston (5) pouvant se déplacer axialement pendant le fonctionnement du moteur à gaz comprimé (1) vers l'espace intérieur arrière (7) par le déplacement du piston (5), au moins un ressort de compression (12) disposé dans l'espace intérieur avant (8), dans lequel l'au moins un ressort de compression (12) prend appui au moins temporairement sur la surface formant base côté avant du cylindre creux (2) pendant le fonctionnement du moteur à gaz comprimé (1), et/ou
au moins un ressort de traction disposé dans l'espace intérieur arrière (7), dans lequel l'au moins un ressort de traction est relié au piston (5) et à la fermeture côté arrière (4) du cylindre creux (2), dans lequel
l'au moins un ressort de compression (12) pousse le piston (5) en direction de la fermeture côté arrière (4) et/ou l'au moins un ressort de traction tire le piston (5) en direction de la fermeture côté arrière (4) jusqu'à ce que l'espace intérieur arrière (7) du cylindre creux (2) soit fermé par le piston (5) par rapport à l'au moins une ouverture d'aération (10) et que l'espace intérieur arrière (7) soit raccordé à l'embouchure de gaz comprimé (9) lorsque la même pression règne dans l'espace intérieur avant (8) du cylindre creux (2) et dans l'espace intérieur arrière (7) du cylindre creux (2), et dans lequel il existe entre le piston (5) et la paroi formant enveloppe (3) du cylindre creux (2) une fente (11) perméable aux gaz par laquelle l'espace intérieur arrière (7) et l'espace intérieur avant (8) sont raccordés entre eux de manière perméable aux gaz.

2. Moteur à gaz comprimé (1) selon la revendication 1, **caractérisé en ce que**
le moteur à gaz comprimé (1) présente une fermeture côté avant (14) de la surface formant base côté avant du cylindre creux (2), dans lequel la fermeture côté avant (14) présente une percée, en particulier une percée axiale centrale, à travers laquelle la tige d'entraînement (6) est guidée, et dans lequel l'espace intérieur avant (8) du cylindre creux (2) est délimité par le piston (5), la paroi intérieure du cylindre creux (2) et la fermeture côté avant (14), dans lequel la fermeture côté avant (14) présente de préférence au moins une ouverture perméable aux gaz.

3. Moteur à gaz comprimé (1) selon la revendication 1 ou 2, **caractérisé en ce que**
le piston (5) sépare le cylindre creux (2) en deux espaces intérieurs séparés l'un de l'autre, de préférence le piston (5) sépare le cylindre creux (2) de manière non étanche aux gaz en deux espaces intérieurs séparés l'un de l'autre.

4. Moteur à gaz comprimé (1) selon l'une des revendications précédentes,
**caractérisé en ce que**
lors de l'injection d'un gaz comprimé (70) sous pression à travers l'au moins une embouchure de gaz comprimé (9) dans l'espace intérieur arrière (7) du cylindre creux (2), le piston (5) provoque un déplacement oscillant de la tige d'entraînement (6) par changement périodique de l'action du gaz comprimé (70) et de l'atmosphère environnante dans l'espace intérieur arrière (7), dans lequel de préférence l'au moins un ressort de compression (12) et/ou l'au moins un ressort de traction sont tendus par le déplacement du piston (5) en direction de la surface formant base avant du cylindre creux (2) entraîné par le gaz comprimé (70) et le piston (5) est poussé par l'au moins un ressort de compression (12) tendu et/ou par l'au moins un ressort de traction tendu en direction de la surface formant base côté arrière lorsque l'espace intérieur arrière (7) est ouvert vers l'atmosphère environnante.

5. Moteur à gaz comprimé (1) selon l'une des revendications précédentes,
**caractérisé en ce que**
le piston (5) possède un diamètre supérieur ou égal à 20 mm, de préférence supérieur ou égal à 30 mm et de manière particulièrement préférée supérieur ou égal à 35 mm.

6. Moteur à gaz comprimé (1) selon l'une des revendications précédentes,
**caractérisé en ce que**
sur le côté avant de l'au moins une tige d'entraînement (6) est disposé un élément de fixation (18), en particulier un filetage, par l'intermédiaire duquel un outil, tel qu'une scie (40, 100), une râpe (102) ou une brosse (104), peut être fixé à l'au moins une tige d'entraînement (6) avec un élément de contre-fixation (44) adapté à l'élément de fixation (18), en particulier un contre-filetage adapté au filetage.

7. Moteur à gaz comprimé (1) selon l'une des revendications précédentes,
**caractérisé en ce que**
la paroi formant enveloppe (3) délimitant le cylindre creux (2), le piston (5), la fermeture côté arrière (4) et, le cas échéant, également une fermeture côté avant (14), sont fabriqués en une matière plastique, en particulier en une matière thermoplastique, de préférence sont formés par moulage par injection.

8. Moteur à gaz comprimé (1) selon l'une des revendications précédentes,
**caractérisé en ce que**
l'au moins un ressort de compression (12) est un ressort en spirale, dans lequel le ressort en spirale entoure la tige d'entraînement (6), dans lequel de préférence aucun ressort de traction n'est disposé dans l'espace intérieur arrière (7).

9. Moteur à gaz comprimé (1) selon l'une des revendications précédentes,
**caractérisé en ce que**
la fente (11) présente une surface de section transversale qui est inférieure à 1 % de la surface de section transversale du cylindre creux (2) perpendiculairement à l'axe de cylindre, de manière particulièrement préférée inférieure à 0,5 %, de manière très particulièrement préférée inférieure à 0,1 % de la surface de section transversale du cylindre creux (2) perpendiculairement à l'axe de cylindre.

10. Moteur à gaz comprimé (1) selon l'une des revendications précédentes,
**caractérisé en ce que**
la position de l'au moins une ouverture d'aération (10) dans la paroi formant enveloppe (3) le long de l'extension axiale du cylindre creux (2) détermine la course du piston (5) avec la tige d'entraînement (6).

11. Système d'entraînement chirurgical présentant un moteur à gaz comprimé (1) selon l'une des revendications précédentes ainsi qu'un élément formant soupape, en particulier un élément formant soupape à actionnement manuel, dans lequel l'élément formant soupape est disposé dans une conduite de gaz comprimé (54) qui est raccordée à l'une de l'au moins une embouchure de gaz comprimé (9) et qui est raccordée ou peut être raccordée à un réservoir de gaz comprimé de sorte que le raccordement au réservoir de gaz comprimé peut être interrompu et/ou la pression au niveau de l'au moins une embouchure de gaz comprimé (9) peut être réglée avec l'élément formant soupape.

12. Système d'entraînement chirurgical selon la revendication 11, **caractérisé en ce que** le système d'entraînement chirurgical présente une poignée (30) avec laquelle il peut tenir dans une main et l'élément formant soupape peut être actionné avec la même main avec une gâchette (34) sur la poignée (30).

13. Système d'entraînement chirurgical selon la revendication 11 ou 12, **caractérisé en ce que** un filtre stérile (60) est disposé dans la conduite de gaz comprimé (54).

14. Système médical de lavage pour le débridement de tissus mous et/ou de tissus osseux présentant un moteur à gaz comprimé (1) selon l'une des revendications 1 à 10 ou un système d'entraînement chirurgical selon l'une des revendications 11 à 13 ou dispositif médical permettant de brosser, râper ou scier des tissus mous et/ou des tissus osseux présentant un moteur à gaz comprimé (1) selon l'une des revendications 1 à 10 ou un système d'entraînement chirurgical selon l'une des revendications 11 à 13, dans lequel un outil, en particulier une scie (40, 100), une râpe (102) ou une brosse (104), est de préférence fixé à la tige d'entraînement (6) du dispositif médical.

15. Procédé permettant de faire fonctionner un moteur à gaz comprimé (1), dans lequel un piston (5) oscille linéairement et axialement dans un cylindre creux (2), dans lequel le piston (5) est relié à une tige d'entraînement (6) qui fait saillie hors du cylindre creux (2) et le piston (5) entraîne la tige d'entraînement (6), dans lequel le procédé comprend les étapes chronologiques suivantes :
A) dans un état initial, délimitation par le piston (5) d'un espace intérieur arrière (7) du cylindre creux (2) qui est fermé par rapport à l'environnement du moteur à gaz comprimé (1), à l'exception d'au moins une embouchure de gaz comprimé (9) ;
B) introduction d'un gaz comprimé (70) dans l'espace intérieur arrière (7) du cylindre creux (2) à travers l'au moins une embouchure de gaz comprimé (9) ;
C) poussée du piston (5) avec la tige d'entraînement (6) en direction d'un côté avant du cylindre creux (2) en agrandissant l'espace intérieur arrière (7) par la pression gazeuse du gaz comprimé (70) dans l'espace intérieur arrière (7) ;
D) tension d'au moins un ressort de compression (12) et/ou d'au moins un ressort de traction par le déplacement du piston (5) ;
E) dégagement d'au moins une ouverture d'aération (10) dans une paroi formant enveloppe (3) du cylindre creux (2) vers l'espace intérieur arrière (7) par le déplacement du piston (5), dans lequel l'au moins une ouverture d'aération (10) est ouverte directement dans l'espace intérieur arrière (7) ;
F) évacuation du gaz comprimé (70) hors de l'espace intérieur arrière (7) à travers l'au moins une ouverture d'aération (10),
G) rappel du piston (5) par une action de force de l'au moins un ressort de compression (12) et/ou de l'au moins un ressort de traction sur le piston (5) ; et
H) fermeture de l'au moins une ouverture d'aération (10) à l'encontre de l'espace intérieur arrière (7) par le déplacement du piston (5), dans lequel
le piston (5) glisse dans le cylindre creux (2) sur un film gazeux du gaz comprimé (70) qui s'écoule à travers une fente (11) entre le piston (5) et la paroi intérieure du cylindre creux (2).

16. Procédé selon la revendication 15, **caractérisé en ce que**
le procédé est mis en oeuvre avec un moteur à gaz comprimé (1) selon l'une des revendications 1 à 10 ou avec un système d'entraînement chirurgical selon l'une des revendications 11 à 13 ou avec un système de lavage ou un dispositif médical permettant de brosser, râper ou scier des tissus mous et/ou des tissus osseux selon la revendication 14.

17. Procédé selon l'une des revendications 15 ou 16, **caractérisé en ce que**
le déplacement de la tige d'entraînement (6) ou du piston (5) entraîne un outil ou une pompe, en particulier entraîne un outil médical, tel qu'une scie (40, 100), une râpe (102) ou une brosse (104), ou un accessoire de lavage (72).
